# EUROPEAN PATENT APPLICATION

(11) **EP 1 956 011 A1**
(43) Date of publication of application: **13.08.2008**
(21) Application number: 06811876.9
(22) Date of filing: 17.10.2006
(51) Int. Cl.: C07D 241/42, C09K 11/06, H01L 51/50

(54) **AMINE COMPOUND AND ORGANIC ELECTROLUMINESCENT ELEMENT EMPLOYING THE SAME**

(30) Priority: 28.11.2005 JP 2005341595
(71) Applicant: Idemitsu Kosan Co., Ltd., Chiyoda-ku Tokyo 100-8321 (JP)
(72) Inventor: INOUE, Tetsuya, Sodegaura-shi Chiba 299-0293 (JP); WATANABE, Masami, Sodegaura-shi Chiba 299-0293 (JP); KONDO, Hirofumi, Sodegaura-shi Chiba 299-0293 (JP)
(74) Representative: Gille Hrabal Struck Neidlein Prop Roos
(86) International application number: PCT/JP2006/320621
(87) International publication number: WO 2007/060795

(57) **Abstract**

The present invention provides an organic electroluminescence device which: brings physical properties, that is, a low ionization potential, large band gap energy, high injection efficiency, and a high mobility into balance in an excellent manner; has high heat resistance; and shows high current efficiency and a long lifetime while maintaining good luminanoe/voltage characteristics and good current density/voltage characteristics, and a novel amine-based compound for realizing the device. The amine-based compound is of a specific structure containing a quinoxaline ring. The organic electroluminescence device includes an organic thin film layer composed of one or more layers including at least a light emitting layer, the organic thin film layer being interposed between a cathode and an anode. In the organic electroluminescence device, at least one layer of the organic thin film layer contains the amine-based compound alone or as a component of a mixture.

## Description

### TECHNICAL FIELD

The present invention relates to an amine-based compound and an organic electroluminescence device using the compound, and more specifically, to an organic electroluminescence device having high heat resistance and showing high current efficiency and a long lifetime while maintaining good luminance/voltage characteristics and good current density/voltage characteristics, and an amine-based compound for realizing the device.

### BACKGROUND ART

An organic electroluminescence (EL) device is a spontaneous light emitting device which utilizes the principle that a fluorescent substance emits light by energy of recombination of holes injected from an anode and electrons injected from a cathode when an electric field is applied thereto. The organic electroluminescence devices are not only highly visible because of the spontaneous light emission, but also excellent in impact strength because they are full solid devices, and thus the organic electroluminescence devices have been attracting attention for the use as a luminescence device in various display devices.
Since an organic EL device of a laminate type driven under a low electric voltage was reported by C. W. Tang et al. of Eastman Kodak Company (C. W. Tang and S. A. Vanslyke, Applied Physics Letters, Volume 51, Pages 913, 1987 or the like), many studies have been conducted on organic EL devices using organic materials as composing materials. Tang et al. used tris(8-quinolinolato)aluminum for a light emitting layer and a triphenyldiamine derivative for a hole transporting layer. Advantages of the laminate structure are that the efficiency of hole injection into the light emitting layer can be increased, that the efficiency of forming excimer which are formed by blocking and recombining electrons injected from the cathode can be increased, and that excimer formed within the light emitting layer can be enclosed. As described above, for the constitution of the organic EL device, a two-layered structure having a hole transporting (injecting) layer and an electron-transporting and light emitting layer and a three-layered structure having a hole transporting (injecting) layer, a light emitting layer and an electron-transporting (injecting) layer are well known. To increase the efficiency of recombination of injected holes and electrons in the devices of the laminate type, the constitution of the device and the process for forming the device have been studied.

Known examples of a hole injecting material for use in the organic EL device include an aromatic amine polymer compound disclosed in Patent Document 1, a triarylamine polymer disclosed in Patent Document 2, and a phenylenediamine derivative disclosed in Patent Document 3.
Since each of those compounds has a small ionization potential, a hole can be easily injected from an anode. Moreover, each of the compounds has a hole mobility higher than that of such star-burst amine derivative as disclosed in Patent Document 4, and is hence suitable as a hole injecting material.
In addition, the materials for use in organic EL devices are roughly classified into a low-molecular-weight-based organic material for producing a device by a vacuum vapor deposition method and a high-molecular-weight-based organic material for producing a device by an application method (such as a spin coating method or an ink-jet method) involving the use of the material in a state of being dissolved in a solution. It should be noted that it is not always necessary for a high-molecular-weight material used here to be a polymer. In general, the material can be used without any problem as long as the material forms an amorphous state at the temperature at which the material is used. Accordingly, a so-called low-molecular-weight amorphous material has been attracting attention.
Examples of known low-molecular-weight-based hole injecting/transporting materials include the following specific compounds. Of the compounds, TPD has been particularly widely used because TPD has an ionization potential as small as 5.4 eV, and shows a high hole mobility. However, the following exemplified compounds each involve, for example, the problem in that each of the compounds has low heat resistance (glass transition temperature, Tg), and crystallizes after a lapse of a long time period even under a room temperature condition so that a film becomes non-uniform.

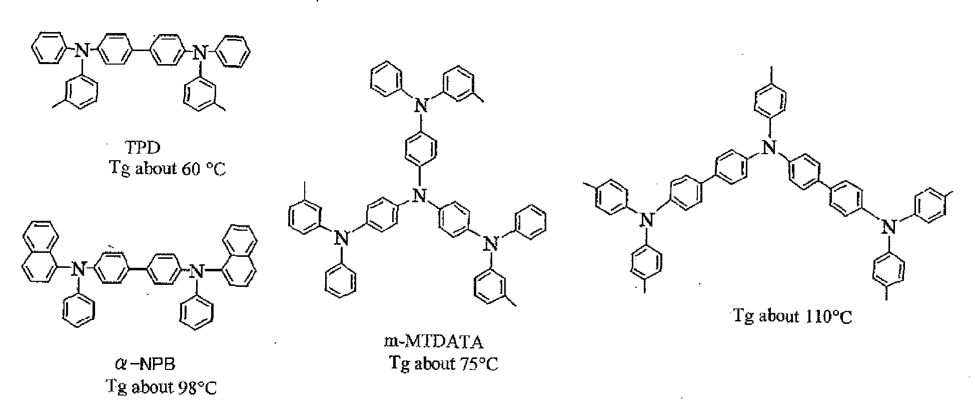

In addition, hole injecting property can be improved by doping a so-called conductive polymer such as a polythiophene-based or polyaniline-based polymer as a high-molecular-weight-based hole injecting/transporting material with an acid such as PSS having the following structure. Research has been also conducted on a high-molecular-weight hole injecting/transporting material having TPD described above incorporated into its main chain or any one of its side chains.
In general, an organic EL device using a high-molecular-weight material is formed by a method that does not need to be performed in a vacuum unlike vacuum deposition. Accordingly, a high-quality thin film is expected to be formed easily from the device, so the device is expected to have a large merit upon production and to be advantageous for an increase in area. However, the device involves a problem of elution due to a solvent upon application of a hole injecting layer, a hole transporting layer, and a light emitting layer forming a laminate structure. Each of PEDOT and PSS having the following structures is an excellent material for use as a hole injecting layer because each of them is water-soluble, and is insoluble in an organic solvent. However, the light emission lifetime of each of them is not always sufficient, and an improvement in the light emission lifetime has been demanded. Potential causes for the insufficient lifetime include the adverse effects of: an oxygen atom produced by the mixing or decomposition of moisture; and a sulfur atom.

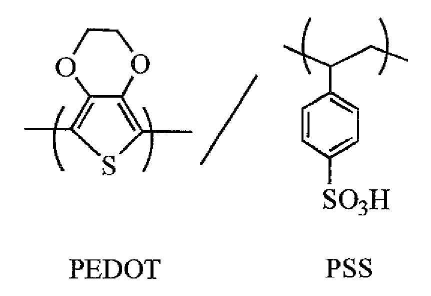

In addition, Patent Document 5 describes a luminescent material for an organic EL device composed of a compound having a quinoxaline skeleton. However, the material has a low glass transition temperature and insufficient heat resistance.
Patent Document 1: JP 9-301934 A
Patent Document 2: WO98/30071 A
Patent Document 3: JP 2000-309566 A
Patent Document 4: JP 4-308688 A
Patent Document 5: JP 2000-053956 A

### DISCLOSURE OF THE INVENTION

### PROBLEMS TO BE SOLVED BY THE INVENTION

The present invention has been made with a view to solving the above-mentioned problems, and an obj ect of the present invention is to provide an organic EL device which: brings physical properties, that is, a low ionization potential, large band gap energy, high injection efficiency, and a highmobility into balance in an excellent manner; has high heat resistance; and shows high current efficiency and a long lifetime while maintaining good luminance/voltage characteristics and good current density/voltage characteristics, and a novel amine-based compound containing a quinoxaline ring for realizing the device.

### MEANS FOR SOLVING THE PROBLEMS

The inventors of the present invention have made extensive studies with a view to achieving the object. As a result, the inventors have found that the glass transition temperature of the compound having a quinoxaline skeleton described in Patent Document 5 described above can be increased by additionally expanding the conjugate structure of the compound, and hence the object can be achieved. Thus, the inventors have completed the present invention.

That is, the present invention provides an amine-based compound containing a quinoxaline ring and represented by the following general formula (1):

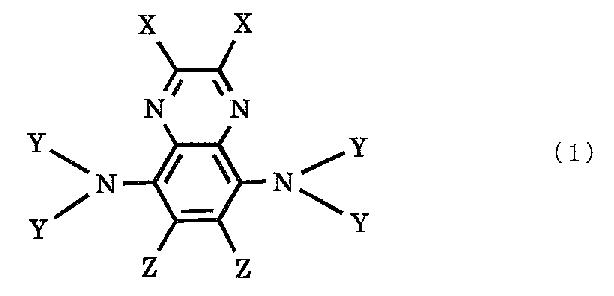

wherein X's each independently represent a hydrogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, the two X's may be identical to or different from each other, at least one X represents any one of the above-mentioned groups except a hydrogen atom, and the two X's crosslink with each other to form a cyclic structure;
Y's each independently represent a substituted or unsubstituted aryl group having 4 to 50 carbon atoms, or a substituted or unsubstituted heterocyclic group having 5 to 50 carbon atoms, and two Y's bonded to the same nitrogen atom may be identical to or different from each other, and may be bonded to and crosslink with each other; and
Z's each independently represent a hydrogen atom, a halogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, and the two Z's may be identical to or different from each other, and may be bonded to and crosslink with each other.

Further, the present invention provides an organic electroluminescence device, including an organic thin film layer composed of one or more layers including at least a light emitting layer, the organic thin film layer being interposed between a cathode and an anode, in which at least one layer of the organic thin film layer contains the amine-based compound containing a quinoxaline ring alone or as a component of a mixture.

### EFFECT OF THE INVENTION

The amine-based compound of the present invention is useful as a hole injecting material or hole transporting material for an organic EL device or for an electrophotographic photosensitive member. An organic EL device using the amine-based compound of the present invention brings physical properties, that is, a low ionization potential, large band gap energy, high injection efficiency, and a high mobility into balance in an excellent manner, has high heat resistance, and shows high current efficiency and a long lifetime while maintaining good luminance/voltage characteristics and good current density/voltage characteristics.

### BEST MODE FOR CARRYING OUT THE INVENTION

An amine-based compound of the present invention is represented by the following general formula (1).

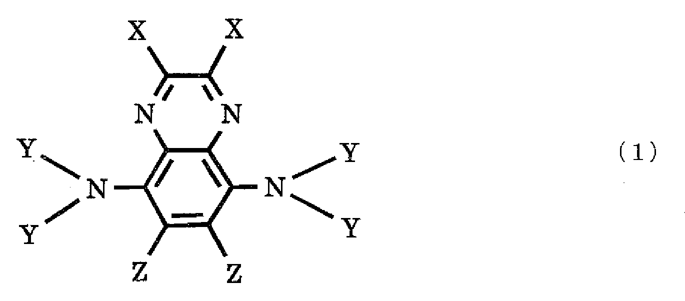

In the general formula (1), X's each independently represent a hydrogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms.

In the general formula (1), the two X's may be identical to or different from each other. At least one X represents any one of the above-mentioned groups except a hydrogen atom, and the two X's crosslink with each other to form a cyclic structure.
Examples of the cyclic structure which is formed by two x's include: cycloalkenes each having 4 to 12 carbon atoms such as cyclobutene, cyclopentene, cyclohexene, cycloheptene, and cyclooctene; cycloalkadienes each having 6 to 12 carbon atoms such as cyclohexadiene, cycloheptadiene, and cyclooctadiene; and aromatic rings each having 6 to 50 carbon atoms such as benzene, naphthalene, phenanthrene, anthracene, pyrene, chrysene, and acenaphthylene; aromatic ring-containing groups each having 6 to 50 carbon atoms such as indene and fluorene; and heterocyclic rings each having 5 to 50 carbon atoms such as imidazole, pyrrole, furan, thiophene, pyridine, benzothiophene, benzofuran, carbazole, dibenzothiophene, and dibenzofuran.
Of those, the aromatic rings each having 6 to 50 carbon atoms such as benzene, naphthalene, phenanthrene, anthracene, pyrene, chrysene, and acenaphthylene are preferable because each of them can increase mobility. Of the aromatic rings, an aromatic ring having 6 to 15 carbon atoms is more preferable because it facilitates vacuum deposition.
In addition, an example of the cyclic structure formed by the two X's is the following general formula (3). When the X's represent a hydrogen atom and a naphthyl group, they crosslink with each other to form the structure shown in the following general formula (3) in which a naphthalene ring and a quinoxaline ring are bonded to each other.

In the general formula (1), Y's each independently represent a substituted or unsubstituted aryl group having 4 to 50 carbon atoms, or a substituted or unsubstituted heterocyclic group having 5 to 50 carbon atoms, and two Y's bonded to the same nitrogen atom may be identical to or different from each other, and may be bonded to and crosslink with each other.
Examples of a cyclic structure that may be formed by the mutual bonding and crosslinking of the two Y's include the following basic skeletons, or substituted bodies of the skeletons.

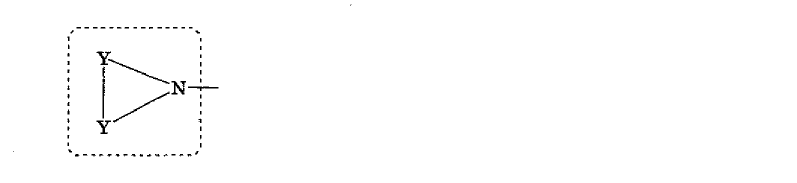

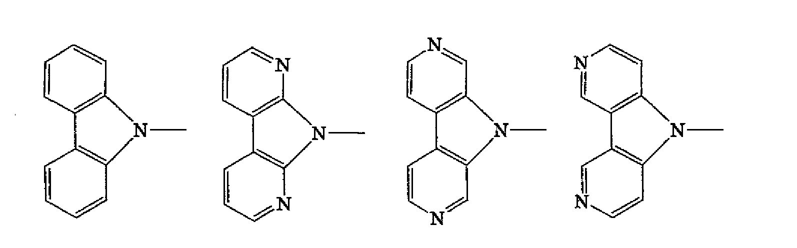

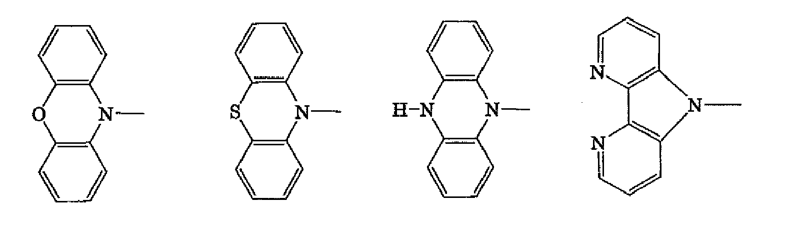

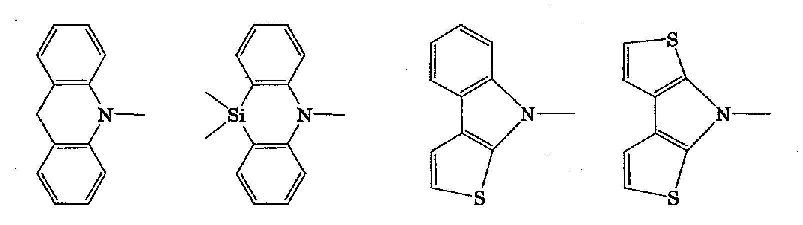

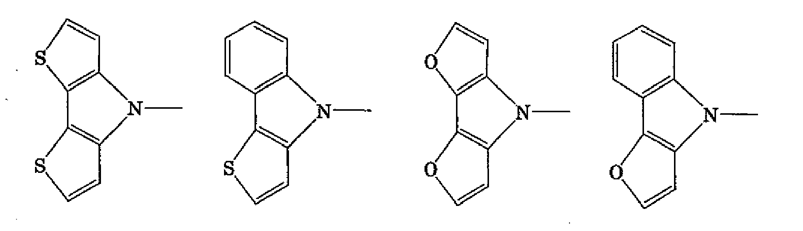

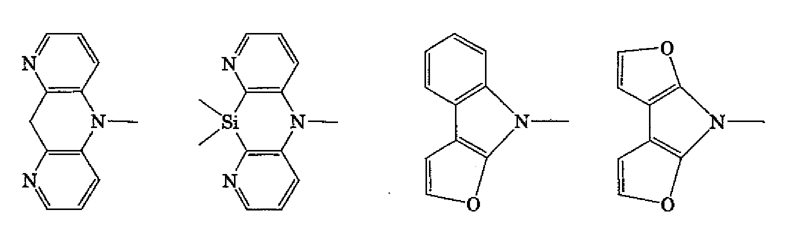

Of those, the following structures are preferable because of the following reason. The following carbazole structure is excellent in electron transporting property, and any other structure is excellent in hole injecting property or hole transporting property because it can lower an ionization potential level by cutting conjugation.

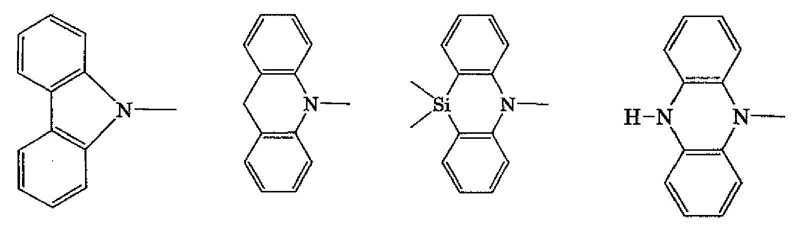

In the general formula (1), Z's each independently represent a hydrogen atom, a halogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms. The two Z's may be identical to or different from each other, and may bond and crosslink with each other.
Examples of a cyclic structure that may be formed by the mutual bonding and crosslinking of the two Z's include examples similar to those of the cyclic structure formed by the two X's described above.

The amine-based compound represented by the general formula (1) is preferably an amine-based compound represented by the following general formula (2) or (3) because of the reasons including the high mobility of the compound, the ease with which the compound is deposited, the easy availability of a raw material for the compound, the ease with which the compound is synthesized, and the good symmetry of a molecule of the compound.

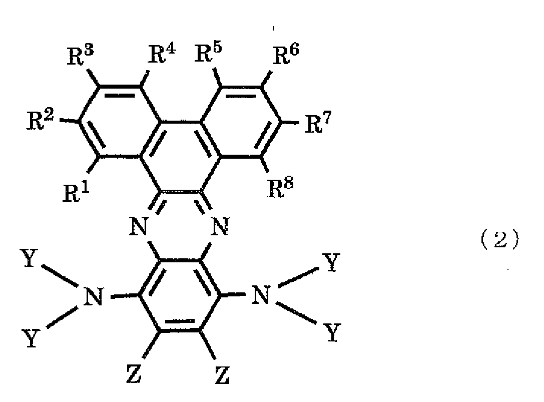

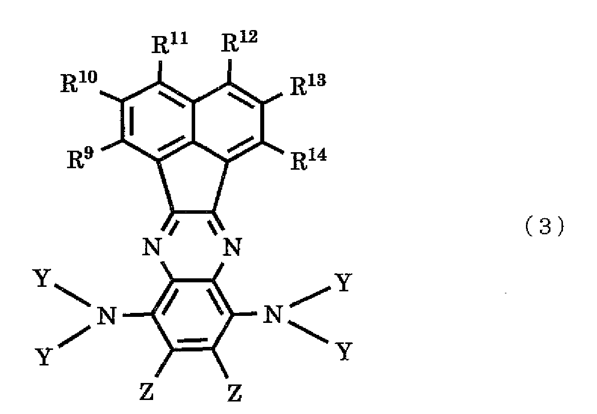

R¹ to R⁸ in the general formula (2) and R⁹ to R¹⁴ in the general formula (3) each independently represent a hydrogen atom, a halogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, and may be identical to or different from one another, and two arbitrary adjacent groups of R¹ to R¹⁴ may crosslink with each other to form a cyclic structure, and examples thereof include the same one as the cyclic structure formed by the two X's described above.

Specific examples of each of the groups represented by X, Y, Z, and R² to R¹⁴ in the general formulae (1) to (3) will be described below.
Examples of the aryl group represented by Y include: a phenyl group, or substituted phenyl groups such as various tolyl group, and various xylyl groups; a 1-naphthyl group, or substituted 1-naphthyl groups such as various methyl substituted 1-naphthyl groups and various dimethyl substituted 1-naphthyl groups; a 2-naphthyl group, or substituted 2-naphthyl groups such as various methyl substituted 2-naphthyl groups and various dimethyl substituted 2-naphthyl groups; a (substituted) 1-anthryl group; a (substituted) 2-anthryl groups; a (substituted) 9-anthryl group; a (substituted) 1-phenanthryl group; a (substituted) 2-phenanthryl group; a (substituted) 3-phenanthryl group; a (substituted) 4-phenanthryl group; a (substituted) 9-phenanthryl group; a (substituted) 1-naphthacenyl group; a (substituted) 2-naphthacenyl group; a (substituted) 9-naphthacenyl group; a (substituted) 1-pyrenyl group; a (substituted) 2-pyrenyl group; and a (substituted) 4-pyrenyl group. Of those, the (substituted) phenyl group and the (substituted) naphthyl group are preferable.
A substituent of each of those groups is not limited to a hydrocarbon group. Examples of the substituent include a hetero atom-containing group such as an alkoxy group, a carboxy group, a carboxy ester group, an aryloxy group, a dialkylamino group, a diarylamino group, or a thioalkoxy group. In addition, an atom which forms nucleus is not limited to a carbon. Examples of the atom may include a hetero atom such as an oxygen atom (e.g. , a furyl group), a nitrogen atom (e.g. , a pyridyl group or a pyrrolyl group), a boron atom (e.g., a boraphenyl group), a silicon atom (e.g., a silaphenyl group), and a sulfur atom (e.g., a thiofuryl group).

Examples of the heterocyclic group represented by Y include a pyridinyl group, a pyradinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a triatinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group, a benzoimidazolyl group, and a purinyl group. In addition, each of those groups may have a substituent, and a substituent may include the same groups as those described in the aryl group.

Examples of the halogen atom represented by each of Z and R¹ to R¹⁴ include fluorine, chlorine, bromine, and iodine.
Examples of the alkyl group represented by each of X, Z, and R¹ to R¹⁴ include a methyl group, an ethyl group, a propyl group, an isopropyl group, an n-butyl group, an s-butyl group, an isobutyl group, a t-butyl group, an n-pentyl group, an n-hexyl group, an n-heptyl group, and an n-octyl group.
Examples of the aryl group represented by each of X, Z, and R¹ to R¹⁴ include: a phenyl group, or substituted phenyl groups such as various tolyl groups and various xylyl groups; a 1-naphthyl group, or substituted 1-naphthyl groups such as various methyl substituted 1-naphthyl groups and various dimethyl substituted 1-naphthyl groups; a 2-naphthyl group, or substituted 2-naphthyl groups such as various methyl substituted 2-naphthyl groups and various dimethyl substituted 2-naphthyl groups; a (substituted) 1-anthryl group; a (substituted) 2-anthryl groups; a (substituted) 9-anthryl group; a (substituted) 1-phenanthryl group; a (substituted) 2-phenanthryl group; a (substituted) 3-phenanthryl group; a (substituted) 4-phenanthryl group; a (substituted) 9-phenanthryl group; a (substituted) 1-naphthacenyl group; a (substituted) 2-naphthacenyl group; a (substituted) 9-naphthacenyl group; a (substituted) 1-pyrenyl group; a (substituted) 2-pyrenyl group; and a (substituted) 4-pyrenyl group.

The alkoxy group represented by each of X, Z, and R¹ to R¹⁴ is denoted by RO-, and examples of R include those described in the alkyl group.
The aryloxy group represented by each of X, Z, and R¹ to R¹⁴ is denoted by R'O-, and examples of R' include those described in the aryl group.
Examples of the thioalkoxy group represented by each of X, Z, and R¹ to R¹⁴ include the groups corresponding to the respective examples described in the alkoxy group.
Examples of the thioaryloxy group represented by each of X, Z, and R¹ to R¹⁴ include the groups corresponding to the respective examples described in the aryloxy group.
Examples of the amino group represented by each of X, Z, and R¹ to R¹⁴ include a diphenylamino group, a ditolylamino group, a dinaphthylamino group, a naphthylphenylamino group, a diethylamino group, a diethylamino group, and dihexylamino group.

Examples of the alkenyl group represented by each of X, Z, and R¹ to R¹⁴ include a vinyl group, an allyl group, a 1-butenyl group, a 2-butenyl group, a 3-butenyl group, a 1,3-butandienyl group, a 1-methylvinyl group, a styryl group, a 2,2-diphenylvinyl group, a 1,2-diphenylvinyl group, a 1-methylallyl group, a 1,1-dimethylallyl group, a 2-methylallyl group, a 1-phenylallyl group, a 2-phenylallyl group, a 3-phenylallyl group, a 3,3-diphenylallyl group, a 1,2-dimethylallyl group, a 1-phenyl-1-butenyl group, and a 3-phenyl-1-butenyl group.
The alkylcarbonyl group represented by each of X, Z, and R¹ to R¹⁴ is denoted by RCO-, and examples of R include those described in the alkyl group.
The arylcarbonyl group represented by each of X, Z, and R¹ to R¹⁴ is denoted by R' CO-, and examples of R' include those described in the aryl group.
Examples of substituents of each of the groups include a substituted or unsubstituted aryl group having 5 to 50 ring carbon atoms, a substituted or unsubstituted alkyl group having 1 to 50 carbon atoms, a substituted or unsubstituted alkoxy group having 1 to 50 carbon atoms, a substituted or unsubstituted aralkyl group having 6 to 50 ring carbon atoms, a substituted or unsubstituted aryloxy group having 5 to 50 ring carbon atoms, a substituted or unsubstituted arylthio group having 5 to 50 ring carbon atoms, a substituted or unsubstituted alkoxycarbonyl group having 1 to 50 carbon atoms, an amino group, a halogen atom, a cyano group, a nitro group, a hydroxyl group, and a carboxyl group.

Specific examples of the amine-based compound of the present invention will be shown below, but the present invention is not limited to those exemplified compounds.

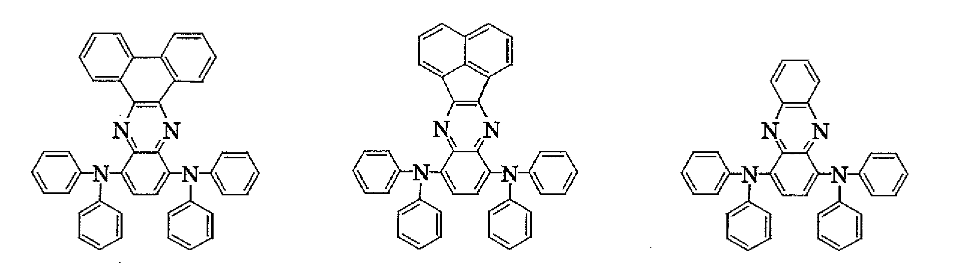

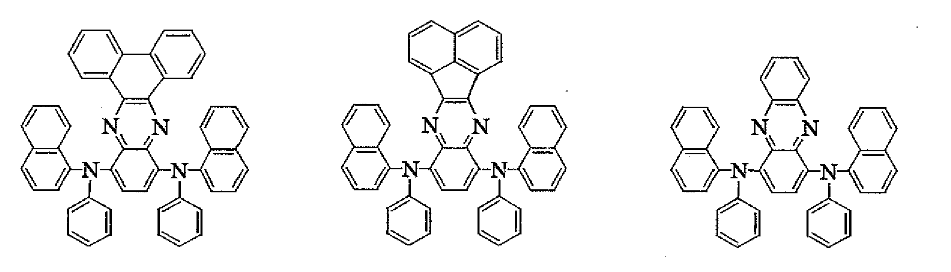

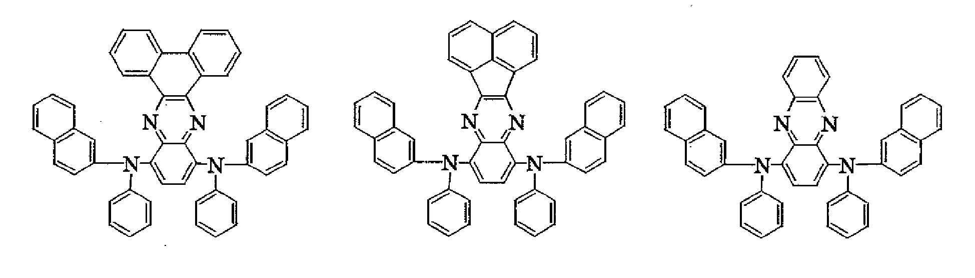

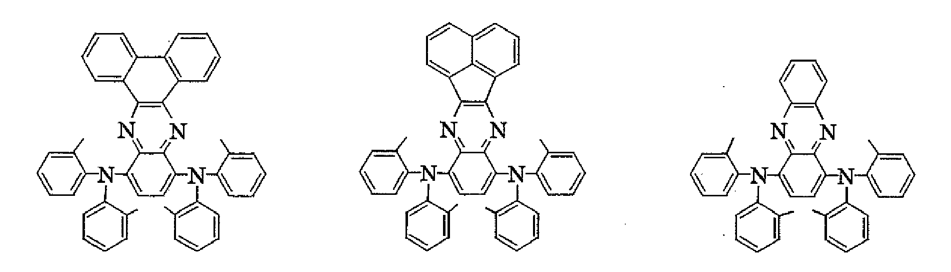

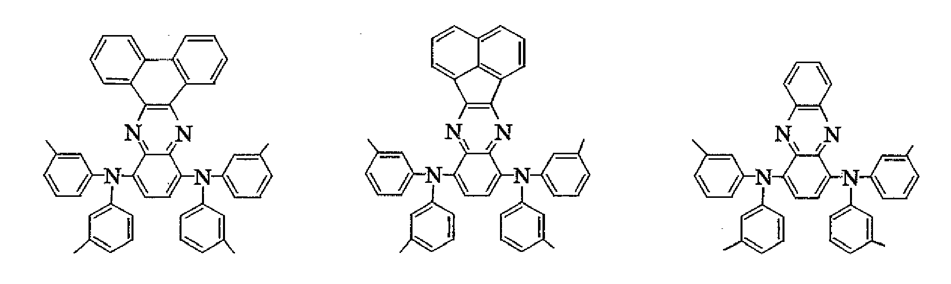

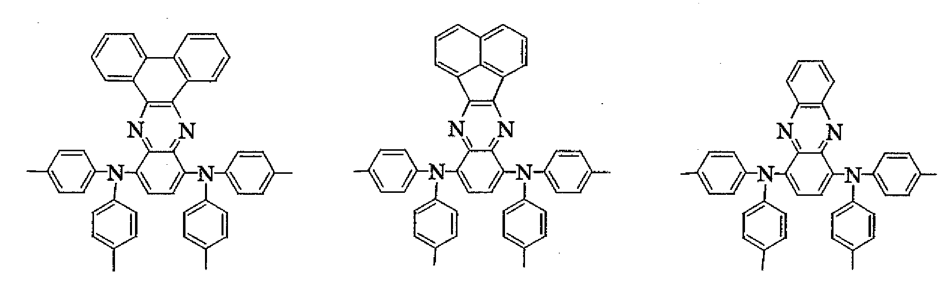

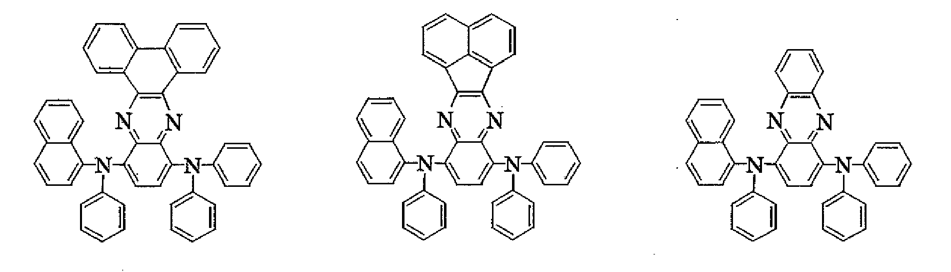

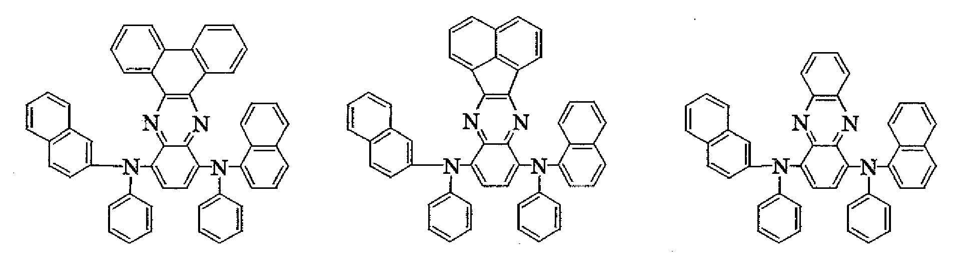

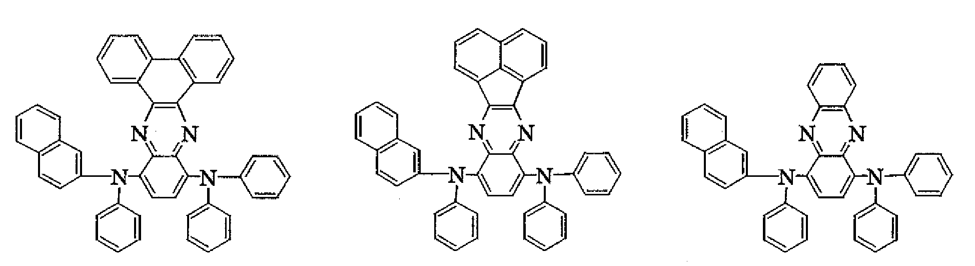

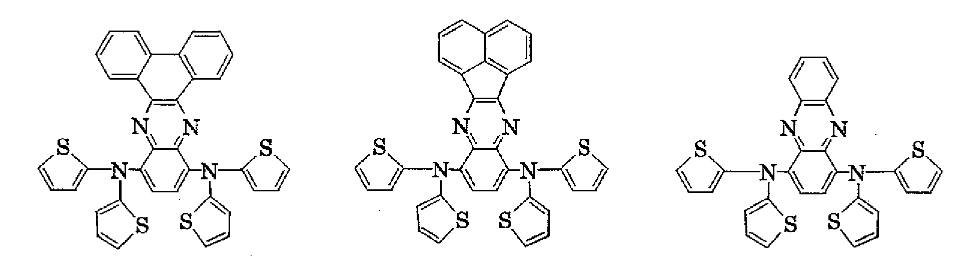

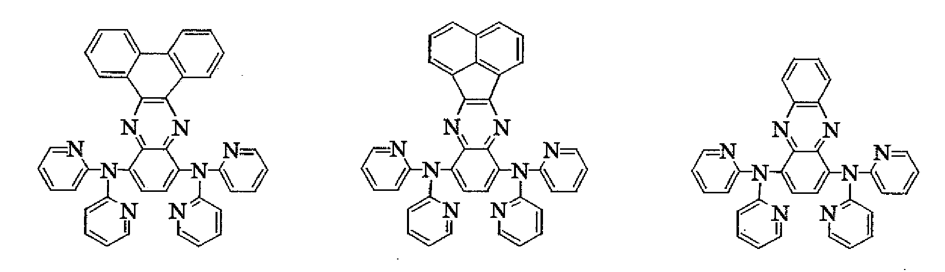

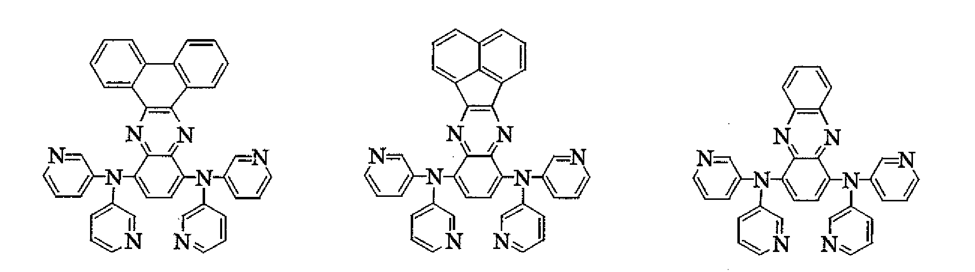

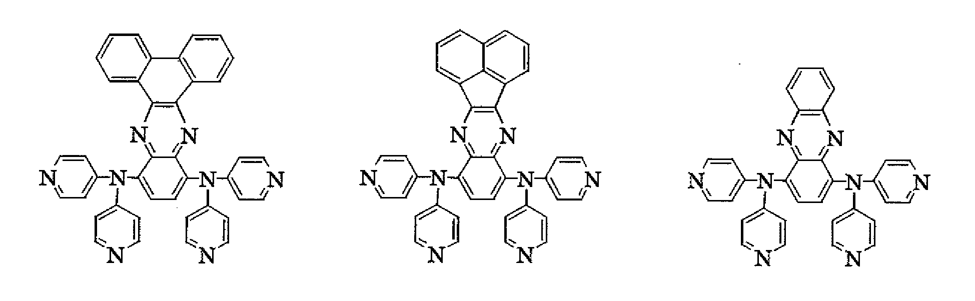

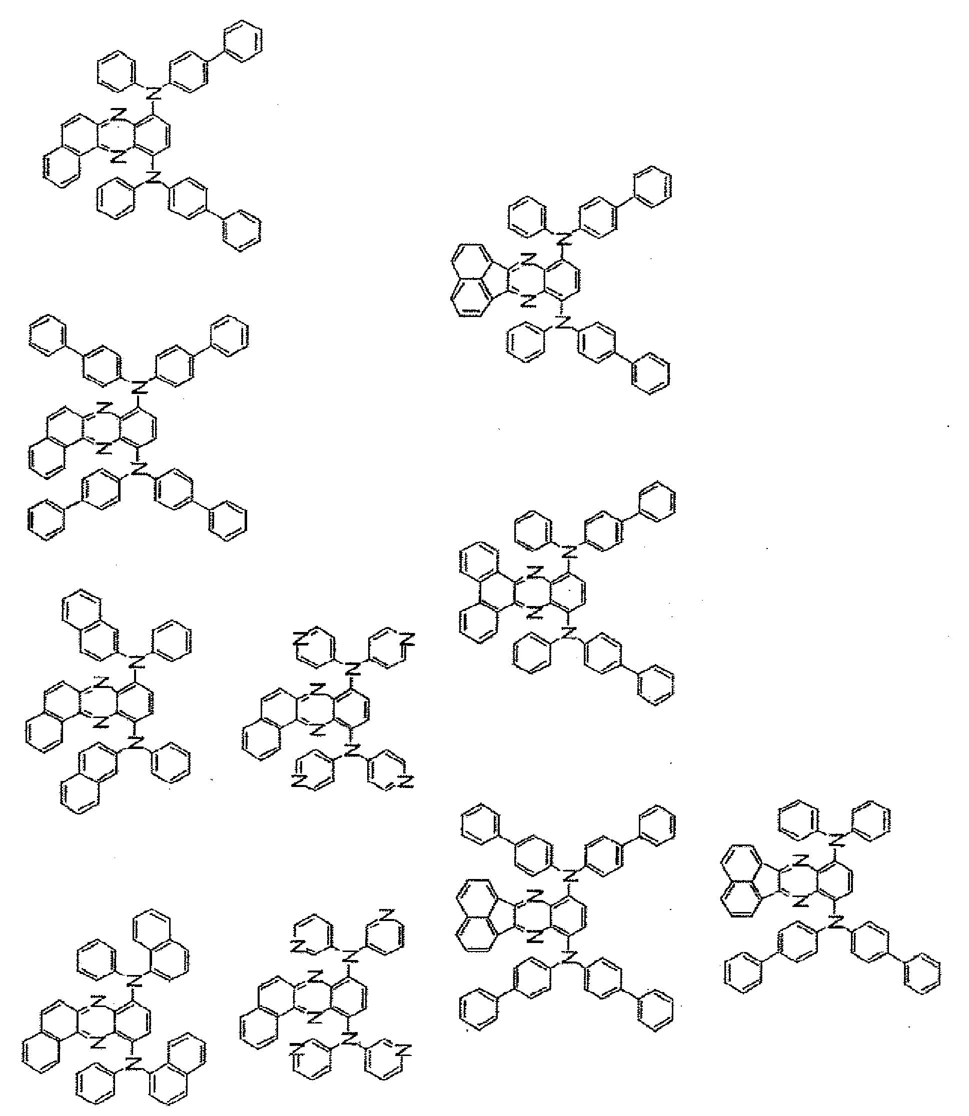

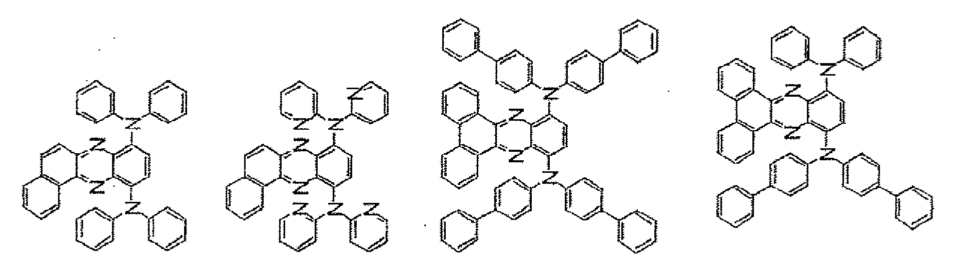

Hereinafter, a method of producing the amine-based compound of the present invention will be described.
The amine-based compound of the present invention can be produced by using a known reaction (reference: for example, Journal of Synthetic Organic Chemistry, Japan, 59-th issue, vol. 6, 2001, p 607). Hereinafter, synthesis methods [1] and [2] for representative compound groups are exemplified. However, a method of producing the amine-based compound is not limited to the following methods.

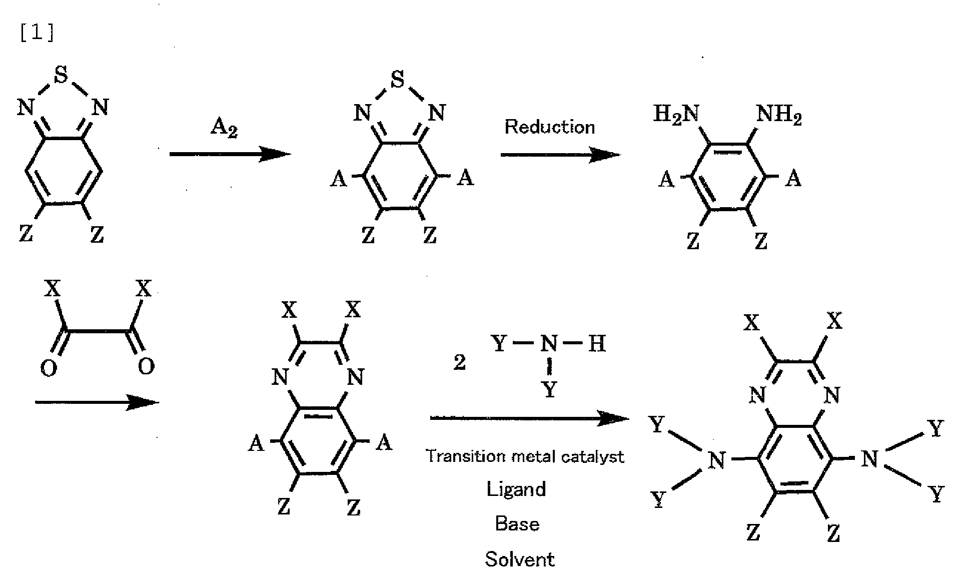

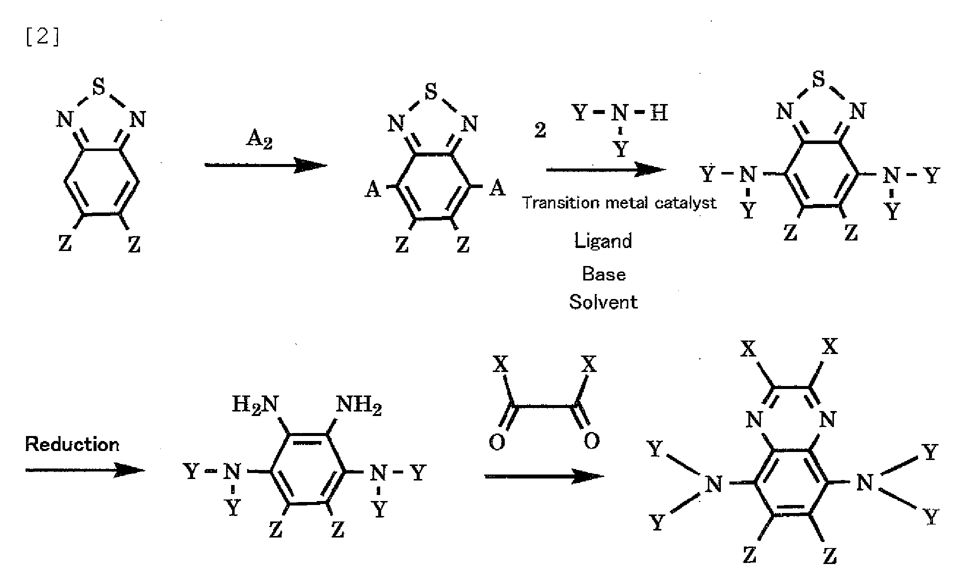

The respective compounds and reagents used in the synthesis methods [1] and [2] will be described.
N's each represent a nitrogen atom, and X's, Y's, and Z's are as described above.
A's each represent a halogen atom, preferably iodine, bromine, or chlorine, or more preferably bromine.
The transition metal catalyst is a transition metal compound containing a metal belonging to Group 8, 9, or 10 in the periodic table, preferably a transition metal compound containing a metal belonging to Group 10, or more preferably a transition metal compound containing palladium. Specific examples of the catalyst include PdCl₂, Pd(OAc)₂ (where Ac represents an acetyl group), Pd₂(dba)₃ (where dba represents dibenzylildenacetone), BINAP (2,2'-bis(diphenylphosphino)-1,1'-binaphthyl), and DPPF (1,1'-bis(diphenylphosphino)ferrocene).
The ligand is a compound containing a material belonging to Group 15 or 16, preferably a compound containing a material belonging to Group 15, or more preferably a compound containing phosphorus. Specific examples of the ligand include: PAr₃'s (where Ar represents an aryl group) such as P(o-Tol)₃ (where Tol represents a tolyl group) and PPh₃ (where Ph represents a phenyl group); and PR₃'s (where R represents an alkyl group) such as PCy₃ (where Cy represents a cyclohexyl group) and P(tBu)₃ (where tBu represents a t-butyl group).
The base is a compound composed of an alkali metal and a conj ugate base, or an alkaline earth metal and a conjugate base, and a base which can dissociate hydrogen of the reactant amines as a proton, may be used. The base is preferably the conjugate base of a basic compound having an acid dissociation constant (25°C, in water) of 18 or more.
The alkali metal is preferably lithium or sodium, and the alkaline earth metal is preferably magnesium.
Examples of the conjugate base of a basic compound having an acid dissociation constant (25°C, in water) of 18 or more include -N(SiR₃)₂, -NR₂, and -OR (where R represents an alkyl group). Preferable examples of the conjugate base include -N(SiMe₃)₂ (where Me represents a methyl group), -N(isopropyl)₂, and -OtBu.
The solvent is not particularly limited unless the transition metal compound, the ligand or the base reacts with the solvent; preferable examples of the solvent include aromatic solvents such as xylenes and toluene.
A reaction temperature is not particularly limited, but is typically in the range of room temperature to 200°C or the boiling point of the solvent, or preferably in the range of room temperature to 120°C.
A reaction time is not particularly limited, but is typically in the range of 1 hour to 150 hours, or preferably in the range of 3 hours to 100 hours.
A reaction ratio "quinoxaline compound : amine : transition metal compound : ligand : base" (molar ratio) is not particularly limited, but is typically 100 : 180 to 300 : 0.1 to 10 : 0.1 to 40 : 150 to 300, or preferably 100 : 190 to 220 : 0.5 to 5 : 1 to 10 : 180 to 250.
The concentration of a reaction liquid is not particularly limited, but is typically in the range of 0.01 to 2 mol/liter, or preferably in the range of 0.1 to 0.2 mol/liter in terms of the concentration of the quinoxaline compound.

The amine-based compound of the present invention is suitable as a hole inj ectingmaterial or a hole transportingmaterial, and can be applied to a wide variety of fields including a solar cell, an electrophotographic photosensitive member, and an organic EL device. The compound is particularly suitable as a hole injecting material or hole transporting material for an organic EL device.
The organic EL device of the present invention includes an organic thin film layer composed of one or more layers including at least a light emitting layer, the organic thin film layer being sandwiched between a cathode and an anode. In the organic EL device, at least one layer of the organic thin film layer contains the amine-based compound alone or as a component of mixture.
In addition, it is preferable that: the organic thin film layer have at least one of a hole injecting layer and a hole transporting layer; and the at least one of the hole injecting layer and the hole transporting layer contain the amine-based compound of the present invention alone or as a component of a mixture.

The constitution of the organic EL device of the present invention will be described in the following.
Typical examples of the constitution of the organic EL device of the present invention include the following:
(1) an anode/light emitting layer/cathode;
(2) an anode/hole injecting layer/light emitting layer/cathode;
(3) an anode/light emitting layer/electron injecting layer/cathode;
(4) an anode/hole injecting layer/light emitting layer/electron injecting layer/cathode;
(5) an anode/organic semiconductor layer/light emitting layer/cathode;
(6) an anode/organic semiconductor layer/electron barrier layer/light emitting layer/cathode;
(7) an anode/organic semiconductor layer/light emitting layer/adhesion improving layer/cathode;
(8) an anode/hole injecting layer/hole transporting layer/light emitting layer/electron injecting layer/cathode;
(9) an anode/insulating layer/light emitting layer/insulating layer/cathode;
(10) an anode/inorganic semiconductor layer/insulating layer/light emitting layer/insulating layer/cathode;
(11) an anode/organic semiconductor layer/insulating layer/light emitting layer/insulating layer/cathode;
(12) an anode/insulating layer/hole injecting layer/hole transporting layer/light emitting layer/ insulating layer/cathode; and
(13) an anode/insulating layer/hole injecting layer/hole transporting layer/light emitting layer/electron injecting layer/cathode.
Of those, the constitution (8) is preferably used in ordinary cases. However, the constitution is not limited to the foregoing.
The amine-based compound of the present invention, which may be used in any one of the above organic layers, is preferably incorporated into a light emitting band or hole transporting band in those components, or is particularly preferably incorporated into a hole injecting layer. The compound is preferably incorporated into the light emitting band because the compound can be an excellent host material. The compound is preferably incorporated into the hole transporting band because the compound can be an excellent material for a hole injecting layer or for a hole transporting layer, and can be a particularly excellent material for a hole injecting layer.
In addition, the organic EL device of the present invention may have a single light emitting layer, or may have multiple light emitting layers. The total content of the amine-based compound of the present invention in the layer or layers is preferably 30 to 100 mol% because current efficiency improves.

The organic EL device is typically produced on a translucent substrate. The translucent substrate is a substrate that supports the organic EL device, and the translucency is desirably 50% or greater within the visible region ranging from 400 to 700 nm. Further, a smooth substrate is preferably used.
For example, a glass sheet, a synthetic resin sheet, or the like is suitably used as such translucent substrate. Examples of the glass sheet include sheets molded of, in particular, a soda lime glass, a barium/strontium-containing glass, a lead glass, an aluminosilicate glass, a borosilicate glass, a barium borosilicate glass, quartz, or the like. In addition, examples of the synthetic resin sheet include sheets each made of a polycarbonate resin, an acrylic resin, a polyethylene terephthalate resin, a polyether sulfide resin, a polysulfone resin, or the like.

Next, the anode serves to inject a hole into the hole transporting layer or the light emitting layer, and an anode having a work function of 4.5 eV or more is effective. Specific examples of an anode material applicable to the present invention include an indium tin oxide alloy (ITO), an indium zinc alloy (IZO), tin oxide (NESA), gold, silver, platinum, and copper. In addition, a material having a small work function is preferably used for the cathode for the purpose of injecting an electron into an electron transporting layer or the light emitting layer.
The anode can be prepared by forming a thin film of the electrode material described above in accordance with a process such as the vapor deposition process and the sputtering process.
When the light emitted from the light emitting layer is extracted from the anode, it is preferable that the anode have a transmittance of the emitted light greater than 10%. It is also preferable that the sheet resistivity of the anode be several hundred Ω/□ or smaller. The thickness of the anode is, in general, selected in the range of 10 nm to 1 µm and preferably in the range of 10 to 200 nm although the preferable range may be different depending on the used material.

The light emitting layer in the organic EL device of the present invention has a combination of the following functions (1) to (3).
(1) The injecting function: the function of injecting holes from the anode or the hole injecting layer and injecting electrons from the cathode or the electron injecting layer when an electric field is applied.
(2) The transporting function: the function of transporting injected charges (i.e., electrons and holes) by the force of the electric field.
(3) The light emitting function: the function of providing the field for recombination of electrons and holes and leading to the emission of light. However, the easiness of injection may be different between holes and electrons and the ability of transportation expressed by the mobility may be different between holes and electrons. It is preferable that either one of the charges be transferred.
For the process for forming the light emitting layer, a known process such as the vapor deposition process, the spin coating process, and the LB process can be used. It is particularly preferable that the light emitting layer be a molecular deposit film.
The molecular deposit film is a thin film formed by deposition of a material compound in the gas phase or a film formed by solidification of a material compound in a solution or in the liquid phase. In general, the molecular deposit film can be distinguished from the thin film formed in accordance with the LB process (i.e., molecular accumulation film) based on the differences in aggregation structure and higher order structure and functional differences caused by those structural differences.
Further, as disclosed in Japanese Patent Application Laid-Open No. S57-51781, the light emitting layer can also be formed by dissolving a binder such as a resin and the material compounds into a solvent to prepare a solution, followed by forming a thin film from the prepared solution by the spin coating process or the like.
In the present invention, where desired, the light emitting layer may include other known light emitting materials than the light emitting material composed of the amine-based compound of the present invention, or a light emitting layer including other known light emitting material may be laminated to the light emitting layer including the light emitting material of the present invention as long as the object of the present invention is not adversely affected.

In addition, in the organic EL device of the present invention, the light emitting layer is preferably a layer that emits blue-based light. It is preferable that: the light emitting layer be a light emitting layer having a maximum luminous wavelength of 450 to 500 nm; and the layer be composed of a host material and a blue-based dopant. This is because the amine-based compound of the present invention serves as, for example, a material for a hole injecting layer suitable for blue-based light emission on the basis of values for its ionization potential level and energy gap.
A styryl derivative, an arylene derivative, or an aromatic amine (amine derivative) is preferably used as the host material in the light emitting layer. This is because any one of them serves as, for example, a host material suitable for blue-based light emission on the basis of values for its ionization potential level and energy gap.
The styryl derivative is particularly preferably at least one kind selected from the group consisting of a distyryl derivative, a tristyryl derivative, a tetrastyryl derivative, and a styrylamine derivative because it emits light in a blue region.
The arylene derivative is particularly preferably an anthracene derivative, especially, a compound having an arylanthracene skeleton because it emits light in a blue region.
The aromatic amine is preferably a compound having 2 to 4 nitrogen atoms each substituted by an aromatic group, or particularly preferably a compound having 2 to 4 nitrogen atoms each substituted by an aromatic group and having at least one alkenyl group because it emits light in a blue region.

Examples of the styryl derivative and the anthracene derivative include compounds represented by the following general formulae [1] to [6], and examples of the aromatic amine include compounds represented by the following general formulae [7] and [8]:

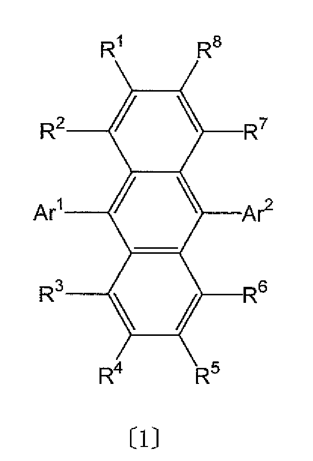

wherein R¹ to R⁸ each independently represent a hydrogen atom, a halogen atom, a cyano group, a nitro group, a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted fused polycyclic group having 10 to 30 carbon atoms, or a substituted or unsubstituted heterocyclic group having 5 to 30 carbon atoms, Ar¹ and Ar² each independently represent a substituted or unsubstituted aryl group having 6 to 30 carbon atoms, or a substituted or unsubstituted alkenyl group, and a substituent is selected from a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted fused polycyclic group having 10 to 30 carbon atoms, and a substituted or unsubstituted heterocyclic group having 5 to 30 carbon atoms;

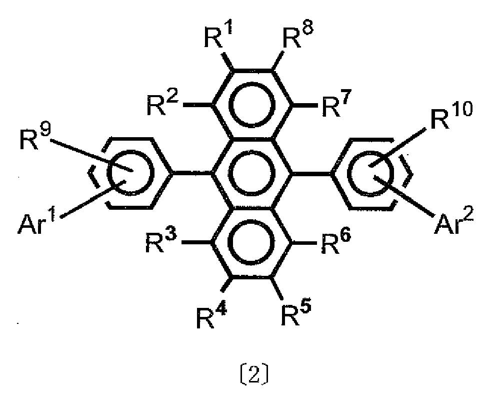

wherein R¹ to R¹⁰ each independently represent a hydrogen atom, a halogen atom, a cyano group, a nitro group, a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted fused polycyclic group having 10 to 30 carbon atoms, or a substituted or unsubstituted heterocyclic group having 5 to 30 carbon atoms, Ar¹ and Ar² each independently represent a substituted or unsubstituted aryl group having 6 to 30 carbon atoms, or a substituted or unsubstituted alkenyl group, and a substituent is selected from a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted fused polycyclic group having 10 to 30 carbon atoms, and a substituted or unsubstituted heterocyclic group having 5 to 30 carbon atoms;

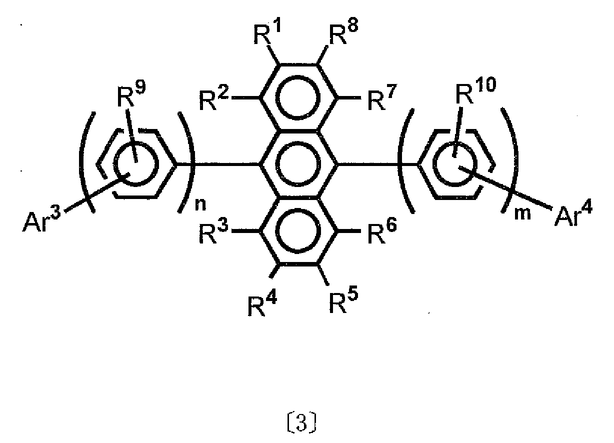

wherein R¹ to R⁸ each independently represent a hydrogen atom, a halogen atom, a cyano group, a nitro group, a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstitutedmonocyclic group having 5 to 30 carbon atoms, a substituted or unsubstituted fused polycyclic group having 10 to 30 carbon atoms, or a substituted or unsubstituted heterocyclic group having 5 to 30 carbon atoms, Ar³ and Ar⁴ each independently represent a substituted or unsubstituted aryl group having 6 to 30 carbon atoms, or a substituted or unsubstituted alkenyl group, and a substituent is selected from a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstitutedmonocyclic group having 5 to 30 carbon atoms, a substituted or unsubstituted fused polycyclic group having 10 to 30 carbon atoms, a substituted or unsubstituted heterocyclic group having 5 to 30 carbon atoms, and a substituted or unsubstituted alkenyl group having 4 to 40 carbon atoms, and m and n each represent 1 to 3, with relationship of n + m ≥ 2 being established;

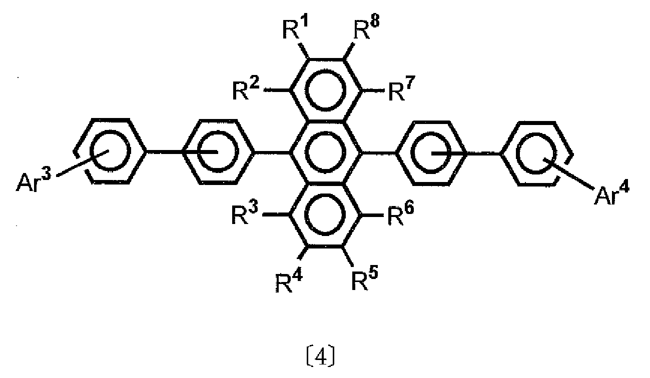

wherein R¹ to R⁸ each independently represent a hydrogen atom, a halogen atom, a cyano group, a nitro group, a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 7 to 30 carbon atoms, an unsubstituted monocyclic group having 5 to 30 carbon atoms, a substituted or unsubstituted fused polycyclic group having 10 to 30 carbon atoms, or a substituted or unsubstituted heterocyclic group having 5 to 30 carbon atoms, Ar³ and Ar⁴ each independently represent a substituted or unsubstituted aryl group having 6 to 30 carbon atoms, or a substituted or unsubstituted alkenyl group, and a substituent is selected from a substituted or unsubstituted alkyl group having 1 to 20 carbon atoms, a substituted or unsubstituted alkoxy group having 1 to 20 carbon atoms, a substituted or unsubstituted aryloxy group having 6 to 30 carbon atoms, a substituted or unsubstituted alkylthio group having 1 to 20 carbon atoms, a substituted or unsubstituted arylthio group having 6 to 30 carbon atoms, a substituted or unsubstituted arylalkyl group having 6 to 30 carbon atoms, an unsubstituted monocyclic group having 5 to 30 carbon atoms, a substituted or unsubstituted fused polycyclic group having 10 to 30 carbon atoms, a substituted or unsubstituted heterocyclic group having 5 to 30 carbon atoms, and a substituted or unsubstituted alkenyl group having 4 to 40 carbon atoms;

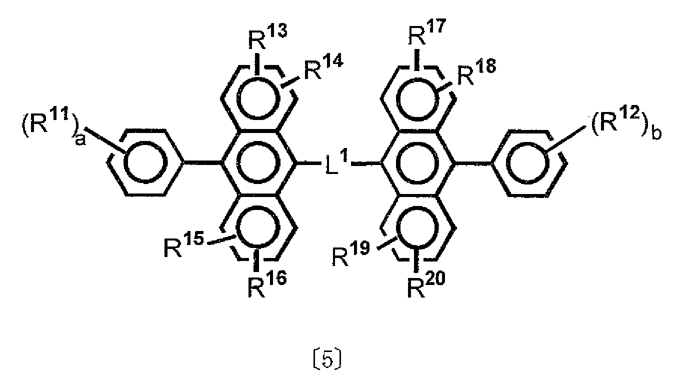

wherein R¹¹ to R²⁰ each independently represent a hydrogen atom, an alkenyl group, an alkyl group, a cycloalkyl group, an aryl group, an alkoxyl group, an aryloxy group, an alkylamino group, an arylamino group, or a heterocyclic group which may be substituted, a and b each represent an integer of 1 to 5, and, when a or b represents 2 or more, R¹¹'s or R¹²'s may be identical to or different from each other, or R¹¹'s or R¹²'s may be bonded to each other to form a ring, R¹³ and R¹⁴, R¹⁵ and R¹⁶, R¹⁷ and R¹⁸, or R¹⁹ and R²⁰ may be bonded to each other to form a ring, and L¹ represents a single bond, -O-, -S-, -N (R) - where R represents an alkyl group or an aryl group which may be substituted, or an arylene group;

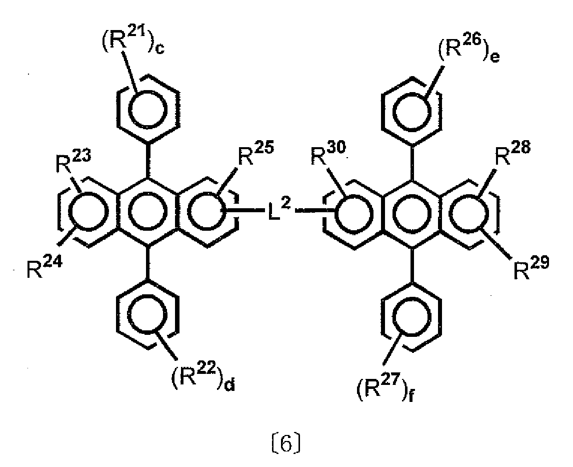

wherein R²¹ to R³⁰ each independently represent a hydrogen atom, an alkenyl group, an alkyl group, a cycloalkyl group, an aryl group, an alkoxyl group, an aryloxy group, an alkylamino group, an arylamino group, or a polycyclic group which may be substituted, c, d, e, and f each represent an integer of 1 to 5, and, when c, d, e, or f represents 2 or more, R²¹'s, R²²'s, R²⁶'s, or R²⁷'s may be identical to or different from each other, or R²¹'s, R²²'s, R²⁶'s, or R²⁷'s may be bonded to each other to form a ring, R²³ and R²⁴, or R²⁸ and R²⁹ may be bonded to each other to form a ring, and L² represents a single bond, -O-, -S-, -N(R)- where R represents an alkyl group or an aryl group which may be substituted, or an arylene group;

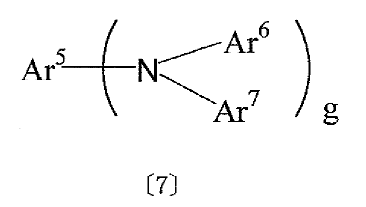

wherein Ar⁵, Ar⁶, and Ar⁷ each independently represent a substituted or unsubstituted, monovalent aromatic group having 6 to 40 carbon atoms, or a styryl group, and g represents an integer of 1 to 4; and

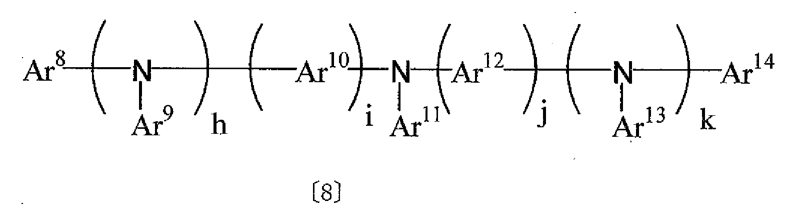

wherein Ar⁸, Ar⁹, Ar¹¹, Ar¹³, and Ar¹⁴ each independently represent a substituted or unsubstituted, monovalent aromatic group having 6 to 40 carbon atoms, or a styryl group, Ar¹⁰ and Ar¹² each independently represent a substituted or unsubstituted, divalent aromatic group having 6 to 40 carbon atoms, or a styrylene group, h and k each represent an integer of 0 to 2, and i and j each represent an integer of 0 to 3.

In addition, the dopant in the light emitting layer is preferably at least one kind selected from amine derivatives such as a styrylamine and an amine-substituted styryl compound, and a fused aromatic ring-containing compound. Examples of the styryl amine and the amine-substituted styryl compound include compounds represented by the following general formulae [9] and [10], and an example of the above fused aromatic ring-containing compound is a compound represented by the following general formula [11]:

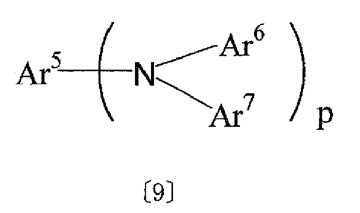

wherein Ar⁵, Ar⁶, and Ar⁷ each independently represent a substituted or unsubstituted aromatic group having 6 to 40 carbon atoms, or a styryl group, and p represents an integer of 1 to 3;

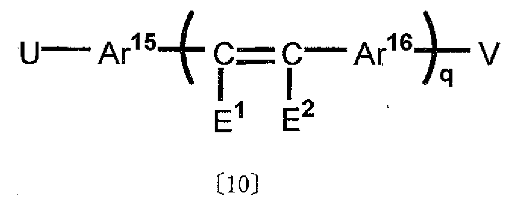

wherein Ar¹⁵ and Ar¹⁶ each independently represent an arylene group having 6 to 30 carbon atoms, E¹ and E² each independently represent an aryl or alkyl group having 6 to 30 carbon atoms, a hydrogen atom, or a cyano group, q represents an integer of 1 to 3, at least one of U and V represents a substituent containing an amino group, and the amino group is preferably an arylamino group; and

(A)ᵣ-B [11]

wherein A represents an alkyl or alkoxy group having 1 to 16 carbon atoms, a substituted or unsubstituted aryl group having 6 to 30 carbon atoms, a substituted or unsubstituted alkylamino group having 6 to 30 carbon atoms, or a substituted or unsubstituted arylamino group having 6 to 30 carbon atoms, B represents a fused aromatic ring group having 10 to 40 carbon atoms, and r represents an integer of 1 to 4.

According to the organic EL device of the present invention, a phosphorescent compound can also be used as a light emitting layer. A compound containing a carbazole ring as a host material is preferable as the phosphorescent compound.
A host material composed of a compound containing a carbazole ring and suitable for phosphorescence is a compound having a function of causing a phosphorescent compound to emit light as a result of the occurrence of energy transfer from the excited state of the host material to the phosphorescent compound. A host compound is not particularly limited as long as it is a compound capable of transferring excimer energy to a phosphorescent compound, and can be appropriately selected in accordance with a purpose. The host compound may have, for example, an arbitrary heterocyclic ring in addition to a carbazole ring.
Specific examples of such a host compound include polymer compounds such as a carbazole derivative, a triazole derivative, an oxazole derivative, an oxadiazole derivative, an imidazole derivative, a polyarylalkane derivative, a pyrazoline derivative, a pyrazolone derivative, a phenylene diamine derivative, an aryl amine derivative, an amino substituted chalcone derivative, a styrylanthracene derivative, a fluorenone derivative, a hydrazone derivative, a stylbene derivative, a silazane derivative, an aromatic tertiary amine compound, a styryl amine compound, an aromatic dimethylidene-based compound, a porphyrin-based compound, an anthraquinodimethane derivative, an anthrone derivative, a diphenylquinone derivative, a thiopyranedioxide derivative, a carbodiimide derivative, a fluorenilidene methane derivative, a distyryl pyrazine derivative, a heterocyclic tetracarboxylic anhydride such as naphthaleneperylene, a phthalocyanine derivative, various metal complex polysilane-based compounds typified by a metal complex of an 8-quinolinol derivative or a metal complex having metal phthalocyanine, benzoxazole, or benzothiazole as a ligand, a poly(N-vinylcarbazole) derivative, an aniline-based copolymer, a conductive high molecular weight oligomer such as a thiophene oligomer or polythiophene, a polythiophene derivative, a polyphenylene derivative, a polyphenylene vinylene derivative, and a polyfluorene derivative. One of the host compounds may be used alone, or two or more of them may be used in combination.

Specific examples thereof include the compounds as described below.

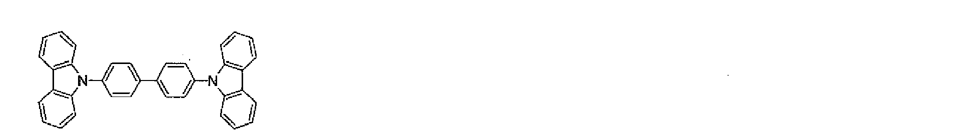

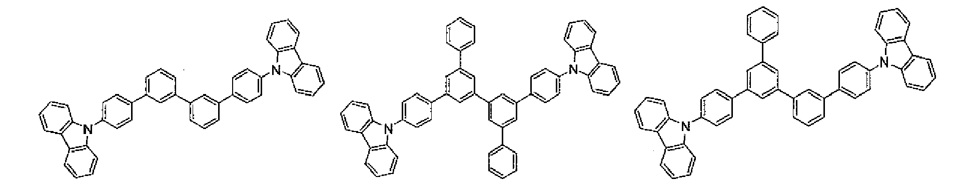

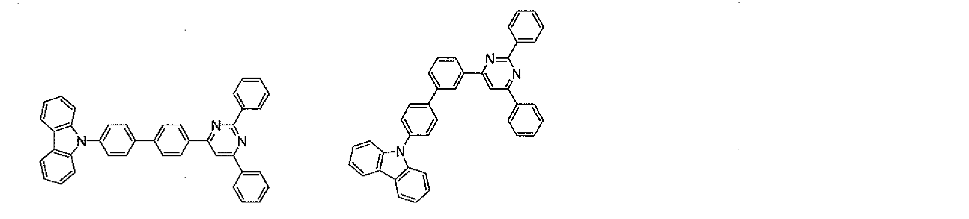

A dopant composed of phosphorescent compound is preferably a compound capable of emitting light from a triplet excimer. The dopant, which is not particularly limited as long as light is emitted from a triplet excimer, is preferably a metal complex containing at least one metal selected from the group consisting of Ir, Ru, Pd, Pt, Os, and Re, and is preferably a porphyrin metal complex or an ortho-metallated metal complex. A porphyrin platinum complex is preferable as the porphyrin metal complex. One kind of a phosphorescent compound may be used alone, or two or more kinds of phosphorescent compounds may be used in combination.
Various ligands can be used for forming an ortho-metallated metal complex. Examples of a preferable ligand include a 2-phenylpyridine derivative, a 7,8-benzoquinoline derivative, a 2-(2-thienyl)pyridine derivative, a 2-(1-naphthyl)pyridine derivative, and a 2-phenylquinoline derivative. Each of those derivatives may have a substituent as required. A fluoride of any one of those derivatives, or one obtained by introducing a trifluoromethyl group into any one of those derivatives is a particularly preferable blue-based dopant. The metal complex may further include a ligand other than the above-mentioned ligands such as acetylacetonate or picric acid as an auxiliary ligand.
The content of the phosphorescent dopant in the light emitting layer is not particularly limited, and can be appropriately selected in accordance with a purpose. The content is, for example, 0.1 to 70 mass%, and is preferably 1 to 30 mass%. When the content of the phosphorescent compound is less than 0.1 mass%, the intensity of emitted light is weak, and an effect of the incorporation of the compound is not sufficiently exhibited. When the content exceeds 70 mass%, a phenomenon referred to as concentration quenching becomes remarkable, and device performance reduces.
In addition, the light emitting layer may contain a hole transporting material, an electron transporting material, or a polymer binder as required.
Further, the thickness of the light emitting layer is preferably 5 to 50 nm, more preferably 7 to 50 nm, or most preferably 10 to 50 nm. When the thickness is 5 nm or greater, it becomes easy to form the light emitting layer and to adjust chromaticity. When the thickness is 50 nm or less, there is no fear that the voltage at which the device is driven may increase.

Next, the hole injecting and transporting layer is a layer which helps injection of holes into the light emitting layer and transports the holes to the light emitting region. The layer exhibits a great mobility of holes and, in general, has an ionization energy as small as 5.5 eV or smaller. For the hole injecting and transporting layer, a material which transports holes to the light emitting layer under an electric field of a smaller strength is preferable. A material which exhibits, for example, a mobility of holes of at least 10⁻⁴ cm²/V·sec under application of an electric field of 10⁴ to 10⁶ V/cm is preferable.
When the amine derivative containing heterocyclic ring of the present invention is used in the hole transporting zone (hole injecting and transporting layer), the compound of the present invention may be used alone or as a mixture with other materials for forming the hole injecting and transporting layer.
The material which can be used for forming the hole injecting and transporting layer as a mixture with the aromatic amine derivative of the present invention is not particularly limited as long as the material has a preferable property described above. The material can be arbitrarily selected from materials which are conventionally used as the charge transporting material of holes in photoconductive materials and known materials which are used for the hole injecting and transporting layer in organic EL devices.

Specific examples include: a triazole derivative (see, for example, US 3,112,197); an oxadiazole derivative (see, for example, US 3, 189, 447); an imidazole derivative (see, for example, JP-B 37-16096); a polyarylalkane derivative (see, for example, US 3,615,402, US 3,820,989, US 3,542,544, JP-B 45-555, JP-B 51-10983, JP-A 51-93224, JP-A 55-17105, JP-A 56-4148, JP-A 55-108667, JP-A 55-156953, and JP-A 56-36656); a pyrazoline derivative and a pyrazolone derivative (see, for example, US 3, 180, 729, US 4, 278, 746, JP-A 55-88064, JP-A 55-88065, JP-A 49-105537, JP-A 55-51086, JP-A 56-80051, JP-A 56-88141, JP-A 57-45545, JP-A 54-112637, and JP-A 55-74546); a phenylenediamine derivative (see, for example, US 3, 615, 404, JP-B 51-10105, JP-B 46-3712, JP-B 47-25336, JP-A 54-53435, JP-A 54-110536, and JP-A 54-119925); an arylamine derivative (see, for example, US 3, 567, 450, US 3, 180, 703, US 3, 240, 597, US 3, 658, 520, US 4, 232, 103, US 4,175,961, US 4,012,376, JP-B 49-35702, JP-B 39-27577, JP-A 55-144250, JP-A 56-119132, JP-A 56-22437, and DE 1,110,518); an amino-substituted chalcone derivative (see, for example, US 3, 526, 501); an oxazole derivative (those disclosed in US 3,257,203); a styrylanthracene derivative (see, for example, JP-A 56-46234); a fluorenone derivative (see, for example, JP-A 54-110837) ; a hydrazone derivative (see, for example, US 3, 717, 462, JP-A 54-59143, JP-A 55-52063, JP-A 55-52064, JP-A 55-46760, JP-A 55-85495, JP-A 57-11350, JP-A 57-148749, and JP-A 2-311591); a stilbene derivative (see, for example, JP-A 61-210363, JP-A 61-228451, JP-A 61-14642, JP-A 61-72255, JP-A 62-47646, JP-A 62-36674, JP-A 62-10652, JP-A 62-30255, JP-A 60-93445, JP-A 60-94462, JP-A 60-174749, and JP-A 60-175052); a silazane derivative (US 4, 950, 950); a polysilane-based copolymer (JP-A 2-204996); an aniline-based copolymer (JP-A 2-282263); and a conductive high molecular weight oligomer (particularly a thiophene oligomer) disclosed in JP-A 1-211399.
In addition to the above-mentioned materials which can be used as the material for the hole injecting layer, a porphyrin compound (those disclosed in, for example, JP-A 63-2956965); an aromatic tertiary amine compound and a styrylamine compound (see, for example, US 4,127,412, JP-A 53-27033, JP-A 54-58445, JP-A 54-149634, JP-A 54-64299, JP-A 55-79450, JP-A 55-144250, JP-A 56-119132, JP-A 61-295558, JP-A 61-98353, and JP-A 63-295695) are preferable, and aromatic tertiary amines are particularly preferable.

Further examples of aromatic tertiary amine compounds include compounds having two fused aromatic rings in the molecule such as 4,4'-bis(N-(1-naphthyl)-N-phenylamino)-biphenyl as disclosed in US 5, 061, 569, and a compound in which three triphenylamine units are bonded together in a star-burst shape, such as 4,4',4"-tris(N-(3-methylphenyl)-N-phenylamino)-triphenylamine as disclosed in JP-A 4-308688.
Further, in addition to the aromatic dimethylidene-based compounds described above as the material for the light emitting layer, inorganic compounds such as Si of the p-type and SiC of the p-type can also be used as the material for the hole injecting layer.
The hole injecting and transporting layer can be formed by forming a thin layer using at least one of the aromatic amine derivative of the present invention and the above-mentioned compound in accordance with a known process such as the vacuum vapor deposition process, the spin coating process, the casting process and the LB process. The thickness of the hole injecting and transporting layer is not particularly limited. In general, the thickness is 5 nm to 5 µm.

Further, an organic semiconductor layer may be disposed as a layer for helping the injection of holes or electrons into the light emitting layer. The organic semiconductor layer having a conductivity of 10⁻¹⁰ S/cm or greater is preferable. Oligomers containing thiophene, and conductive oligomers such as oligomers containing arylamino and conductive dendrimers such as dendrimers containing arylamine which are disclosed in JP-A 08-193191 can be used as the material for the organic semiconductor layer.

Next, the electron injecting and transporting layer is a layer which helps injection of electrons into the light emitting layer, transports the electrons to the light emitting region, and exhibits a great mobility of electrons.
In addition, it is known that, in an organic EL device, emitted light is reflected by an electrode (cathode in this case), so emitted light directly extracted from an anode and emitted light extracted via the reflection by the electrode interfere with each other. The thickness of an electron transporting layer is appropriately selected from the range of several nanometers to several micrometers in order that the interference effect may be effectively utilized. Particularly, when the thickness is large, an electron mobility is preferably at least 10⁻⁵ cm²/Vs or more upon application of an electric field of 10⁴ to 10⁶ V/cm in order to avoid an increase in voltage.
A metal complex of 8-hydroxyquinoline or of a derivative of 8-hydroxyquinoline, or an oxadiazole derivative is suitable as a material to be used for an electron injecting layer. Specific examples of the metal complex of 8-hydroxyquinoline or of a derivative of 8-hydroxyquinoline that can be used as an electron injecting material include metal chelate oxynoid compounds each containing a chelate of oxine (generally 8-quinolinol or 8-hydroxyquinoline) such as tris(8-quinolinol)aluminum.

On the other hand, examples of the oxadiazole derivative include electron transfer compounds represented by the following general formulae: 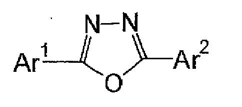 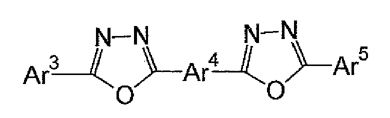 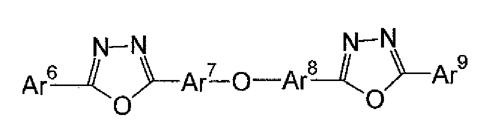 wherein: Ar¹, Ar², Ar³, Ar⁵, Ar⁶ and Ar⁹ each represent a substituted or unsubstituted aryl group and may represent the same group or different groups. Ar⁴, Ar⁷ and Ar⁸ each represent a substituted or unsubstituted arylene group and may represent the same group or different groups.
Examples of the aryl group include a phenyl group, a biphenyl group, an anthranyl group, a perylenyl group, and a pyrenyl group. Examples of the arylene group include a phenylene group, a naphthylene group, a biphenylene group, an anthranylene group, a perylenylene group, and a pyrenylene group. Examples of the substituent include alkyl groups having 1 to 10 carbon atoms, alkoxyl groups having 1 to 10 carbon atoms, and a cyano group. As the electron transfer compound, compounds which can form thin films are preferable.

Examples of the electron transfer compounds described above include the following. 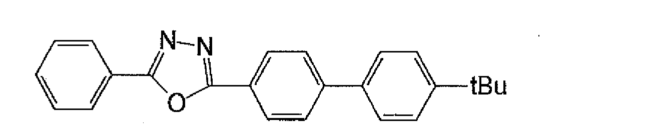 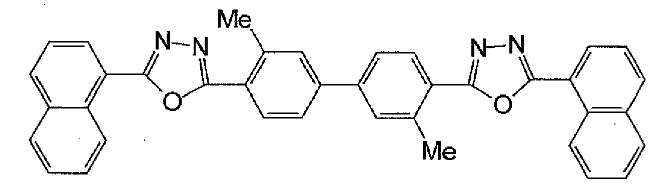 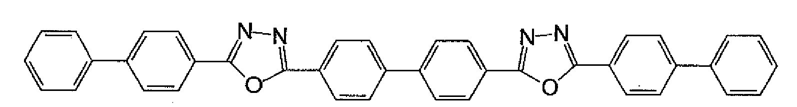 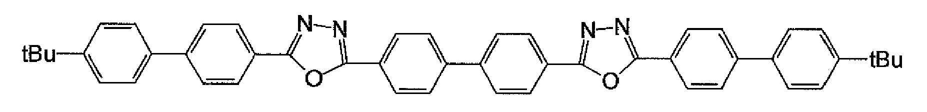 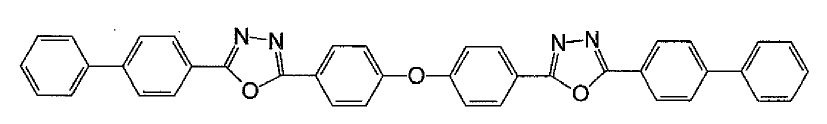 Further, materials represented by the following general formulae (A) to (F) can be used for an electron injecting layer and an electron transporting layer.
A nitrogen-containing heterocycle derivative represented by

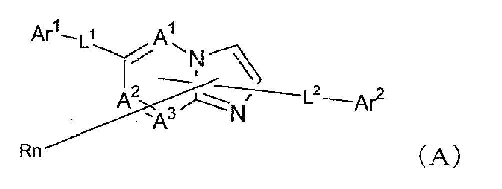

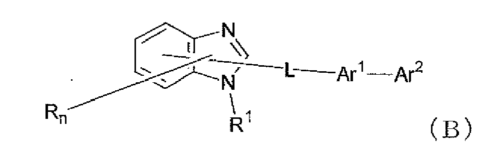

wherein: A¹ to A³ each independently represent a nitrogen atom or a carbon atom;
Ar¹ represents a substituted or unsubstituted aryl group having 6 to 60 ring carbon atoms, or a substitutedor unsubstitutedheteroaryl group having 3 to 60 ring carbon atoms, Ar² represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms, or a divalent group of any one of them provided that one of Ar¹ and Ar² represents a substituted or unsubstituted fused ring group having 10 to 60 ring carbon atoms or a substituted or unsubstituted monohetero fused ring group having 3 to 60 ring carbon atoms;
L¹, L², and L each independently represent a single bond, a substituted or unsubstituted arylene group having 6 to 60 ring carbon atoms, a substituted or unsubstituted heteroarylene group having 3 to 60 ring carbon atoms, or a substituted or unsubstituted fluorenylene group;
R represents a hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, or a substituted or unsubstituted alkoxy group having 1 to 20 carbon atoms. n represents an integer of 0 to 5, and, when n represents 2 or more, multiple R's may be identical to or different from each other, and multiple R groups adjacent to each other may be bonded to each other to form a carbocyclic aliphatic ring or a carbocyclic aromatic ring.

A nitrogen-containing heterocyclic ring derivative represented by HAr-L-Ar¹-Ar² (C)
wherein: HAr represents a nitrogen-containing heterocyclic ring having 3 to 40 carbon atoms which may have a substituent, L represents a single bond, an arylene group having 6 to 60 carbon atoms which may have a substituent, a heteroarylene group having 3 to 60 carbon atoms which may have a substituent, or a fluorenylene group which may have a substituent, Ar¹ represents a divalent aromatic hydrocarbon group having 6 to 60 carbon atoms which may have a substituent, and Ar² represents an aryl group having 6 to 60 carbon atoms which may have a substituent, or a heteroaryl group having 3 to 60 carbon atoms which may have a substituent.

A silacyclopentadiene derivative represented by

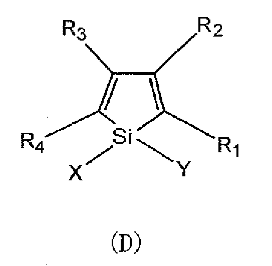

wherein: X and Y each independently represent a saturated or unsaturated hydrocarbon group having 1 to 6 carbon atoms, an alkoxy group, an alkenyloxy group, an alkynyloxy group, a hydroxy group, a substituted or unsubstituted aryl group, or a substituted or unsubstituted heterocycle, or X and Y are bonded to each other to form a structure as a saturated or unsaturated ring; and R₁ to R₄ each independently represent hydrogen, a halogen atom, a substituted or unsubstituted alkyl group having 1 to 6 carbon atoms, an alkoxy group, an aryloxy group, a perfluroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, or, when two or more of R₁ to R₄ are adjacent to each other, they form a structure in which a substituted or unsubstituted ring is condensed.

A borane derivative represented by

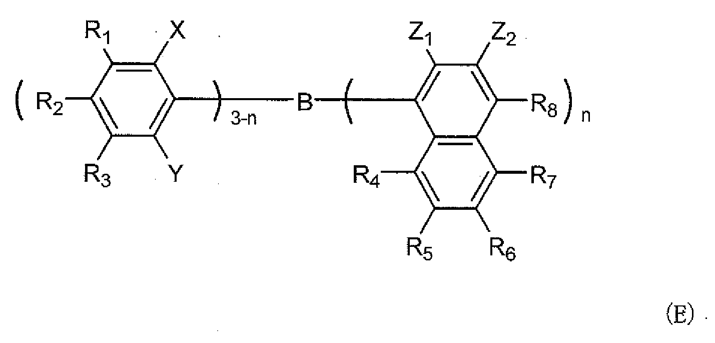

wherein: R₁ to R₈ and Z₂ each independently represent a hydrogen atom, a saturated or unsaturated hydrocarbon group, an aromatic group, a heterocyclic group, a substituted amino group, a substituted boryl group, an alkoxy group, or an aryloxy group; X, Y, and Z₁ each independently represent a saturated or unsaturated hydrocarbon group, an aromatic group, a heterocyclic group, a substituted amino group, an alkoxy group, or an aryloxy group; substituents of Z₁ and Z₂ may be bonded to each other to form a fused ring; and n represents an integer of 1 to 3, and, when n represents 2 or more, Z₁'s may be different from each other provided that the case where n represents 1, X, Y, R₂ each represent a methyl group, and R₈ represents a hydrogen atom or a substituted boryl group and the case where n represents 3 and Z₁'s each represent a methyl group are excluded.

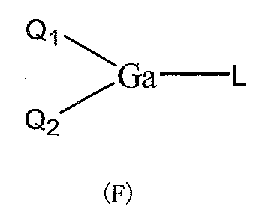

wherein: Q¹ and Q² each independently represent a ligand represented by the following general formula (G) ; and L represents a halogen atom, a substituted or unsubstituted alkyl group, a substituted or unsubstituted cycloalkyl group, a substituted or unsubstituted aryl group, a substituted or unsubstituted heterocyclic group, -OR¹ where R¹ represents a hydrogen atom, a substituted or unsubstituted alkyl group, a substituted or unsubstituted cycloalkyl group, a substituted or unsubstituted aryl group, or a substituted or unsubstituted heterocyclic group, or a ligand represented by -O-Ga-Q³(Q⁴) where Q³ and Q⁴ are identical to Q¹ and Q².

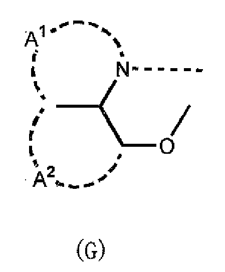

wherein rings A¹ and A² are six-membered aryl ring structures which are condensed with each other and each of which may have a substituent.

The metal complex behaves strongly as an n-type semiconductor, and has a large electron injecting ability. Further, generation energy upon formation of the complex is low. As a result, the metal and the ligand of the formed metal complex are bonded to each other so strongly that the fluorescent quantum efficiency of the complex as a light emitting material increases.
Specific examples of a substituent in the rings A¹ and A² each forming a ligand in the general formula (G) include: a halogen atom such as chlorine, bromine, iodine, or fluorine; a substituted or unsubstituted alkyl group such as a methyl group, an ethyl group, a propyl group, a butyl group, an s-butyl group, a t-butyl group, a pentyl group, a hexyl group, a heptyl group, an octyl group, a stearyl group, or trichloromethyl group; a substituted or unsubstituted aryl group such as a phenyl group, a naphthyl group, a 3-methylphenyl group, a 3-methoxyphenyl group, a 3-fluorophenyl group, a 3-trichloromethylphenyl group, a 3-trifluoromethylphenyl group, or a 3-nitrophenyl group; a substituted or unsubstituted alkoxy group such as a methoxy group, an n-butoxy group, a t-butoxy group, a trichloromethoxy group, a trifluoroethoxy group, a pentafluoropropoxy group, a 2,2,3,3-tetrafluoropropoxy group, an 1,1,1,3,3,3-hexafluoro-2-propoxy group, or a 6-(perfluoroethyl)hexyloxy group; a substituted or unsubstituted aryloxy group such as a phenoxy group, a p-nitrophenoxy group, p-t-butylphenoxy group, a 3-fluorophenoxy group, a pentafluorophenyl group, or a 3-trifluoromethylphenoxy group; a substituted or unsubstituted alkylthio group such as a methylthio group, an ethylthio group, a t-butylthio group, a hexylthio group, an octylthio group, or a trifluoromethylthio group; a substituted or unsubstituted arylthio group such as a phenylthio group, a p-nitrophenylthio group, a p-t-butylphenylthio group, a 3-fluorophenylthio group, a pentafluorophenylthio group, or a 3-trifluoromethylphenylthio group; a mono-substituted or di-substituted amino group such as a cyano group, a nitro group, an amino group, a methylamino group, a diethylamino group, an ethylamino group, a diethylamino group, a dipropylamino group, a dibutylamino group, or a diphenylamino group; an acylamino group such as a bis (acetoxymethyl) amino group, a bis (acetoxyethyl)amino group,a bis(acetoxypropyl)amino group, or a bis (acetoxybutyl) amino group; a carbamoyl group such as a hydroxyl group, a siloxy group, an acyl group, a methylcarbamoyl group, a dimethylcarbamoyl group, an ethylcarbamoyl group, a diethylcarbamoyl group, apropylcarbamoyl group, a butylcarbamoyl group, or a phenylcarbamoyl group; a cycloalkyl group such as a carboxylic acid group, a sulfonic acid group, an imide group, a cyclopentane group, or a cyclohexyl group; an aryl group such as a phenyl group, a naphthyl group, a biphenyl group, an anthranyl group, a phenanthryl group, a fluorenyl group, or a pyrenyl group; and a heterocyclic group such as a pyridinyl group, a pyrazinyl group, a pyrimidinyl group, a pyridazinyl group, a triazinyl group, an indolinyl group, a quinolinyl group, an acridinyl group, a pyrrolidinyl group, a dioxanyl group, a piperidinyl group, a morpholidinyl group, a piperazinyl group, a triatinyl group, a carbazolyl group, a furanyl group, a thiophenyl group, an oxazolyl group, an oxadiazolyl group, a benzoxazolyl group, a thiazolyl group, a thiadiazolyl group, a benzothiazolyl group, a triazolyl group, an imidazolyl group, a benzoimidazolyl group, or a purinyl group. In addition, the above-mentioned substituents may be bound to each other to further form a six-membered aryl ring or a heterocycle.

A preferable embodiment of the organic EL device of the present invention includes a device including a reducing dopant in the region of electron transport or in the interfacial region of the cathode and the organic layer. The reducing dopant is defined as a substance which can reduce a compound having the electron-transporting property. Various compounds can be used as the reducing dopant as long as the compounds have certain reductive property. For example, at least one substance selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, organic complexes of alkali metals, organic complexes of alkaline earth metals, and organic complexes of rare earth metals can be preferably used.
More specifically, examples of the particularly preferable reducing dopant include substances having a work function of 2.9 eV or smaller, specific examples of which include at least one alkali metal selected from the group consisting of Na (the work function: 2.36 eV), K (the work function: 2.28 eV), Rb (the work function: 2.16 eV), and Cs (the work function: 1. 95 eV) and at least one alkaline earth metal selected from the group consisting of Ca (the work function: 2.9 eV), Sr (the work function: 2.0 to 2.5 eV), and Ba (the work function: 2. 52 eV). Among the above-mentioned substances, at least one alkali metal selected from the group consisting of K, Rb, and Cs is more preferable, Rb and Cs are still more preferable, and Cs is most preferable as the reducing dopant. Those alkali metals have great reducing ability, and the luminance of the emitted light and the life time of the organic EL device can be increased by addition of a relatively small amount of the alkali metal into the electron injecting zone. As the reducing dopant having a work function of 2. 9 eV or smaller, combinations of two or more alkali metals thereof are also preferable. Combinations having Cs such as the combinations of Cs and Na, Cs and K, Cs and Rb, and Cs, Na, and K are more preferable. The reducing ability can be efficiently exhibited by the combination having Cs. The luminance of emitted light and the life time of the organic EL device can be increased by adding the combination having Cs into the electron injecting zone.

The present invention may further include an electron injecting layer which is composed of an insulating material or a semiconductor and disposed between the cathode and the organic layer. At this time, leak of electric current can be effectively prevented by the electron injecting layer and the electron injecting property can be improved. As the insulating material, at least one metal compound selected from the group consisting of alkali metal chalcogenides, alkaline earth metal chalcogenides, alkali metal halides, and alkaline earth metal halides is preferable. It is preferable that the electron injecting layer be composed of the above-mentioned substance such as the alkali metal chalcogenide since the electron injecting property can be further improved. Specifically, preferable examples of the alkali metal chalcogenide include Li₂O, K₂O, Na₂S, Na₂Se, and Na₂O, and preferable examples of the alkaline earth metal chalcogenide include CaO, BaO, SrO, BeO, BaS, and CaSe. Preferable examples of the alkali metal halide include LiF, NaF, KF, LiCl, KCl, and NaCl. Preferable examples of the alkaline earth metal halide include fluorides such as CaF₂, BaF₂, SrF₂, MgF₂, and BeF₂ and halides other than the fluorides.
Examples of the semiconductor composing the electron-transporting layer include oxides, nitrides, and oxynitrides of at least one element selected from Ba, Ca, Sr, Yb, Al, Ga, In, Li, Na, Cd, Mg, Si, Ta, Sb, and Zn used alone or in combination of two or more. It is preferable that the inorganic compound composing the electron-transporting layer form a fine crystalline or amorphous insulating thin film. When the electron injecting layer is composed of the insulating thin film described above, a more uniform thin film can be formed, and defects of pixels such as dark spots can be decreased. Examples of the inorganic compound include alkali metal chalcogenides, alkaline earth metal chalcogenides, alkali metal halides, and alkaline earth metal halides which are described above.

Next, a material such as a metal, an alloy, a conductive compound, or a mixture of those materials which has a small work function (4 eV or smaller) is used as the cathode. Specific examples of the electrode material include sodium, sodium-potassium alloys, magnesium, lithium, magnesium-silver alloys, aluminum/aluminum oxide, Al/Li₂O, Al/LiO₂, Al/LiF, aluminum-lithium alloys, indium, and rare earth metals.
The cathode can be prepared by forming a thin film of the electrode material described above in accordance with a process such as the vapor deposition process or the sputtering process.
When the light emitted from the light emitting layer is obtained through the cathode, it is preferable that the cathode has a transmittance of the emitted light greater than 10%. It is also preferable that the sheet resistivity of the cathode is several hundred Ω/□ or smaller. The thickness of the cathode is, in general, selected in the range of 10 nm to 1 µm and preferably in the range of 50 to 200 nm.

Further, in general, defects in pixels tend to be formed in organic EL device due to leak and short circuit since an electric field is applied to ultra-thin films. To prevent the formation of the defects, a layer of a thin film having an insulating property is preferably inserted between the pair of electrodes.
Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide. Mixtures and laminates of the above-mentioned compounds may also be used.

Next, regarding a method of preparing the organic EL device of the present invention, the anode and the light emitting layer, and when necessary, the hole injecting and the transporting layer and the electron injecting and transporting layer are formed in accordance with the above-mentioned process using the above-mentioned materials, and the cathode may be formed in the last step. The organic EL device may also be prepared by forming the above-mentioned layers in the order reverse to that described above, i.e., the cathode being formed in the first step and the anode in the last step.
Hereinafter, an embodiment of the process for preparing an organic EL device having a construction in which an anode, a hole injecting layer, a light emitting layer, an electron injecting layer, and a cathode are disposed successively on a substrate transmitting light will be described.
On a suitable translucent substrate, a thin film made of a material for the anode is formed in accordance with the vapor deposition process or the sputtering process so that the thickness of the formed thin film is 1 µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process, or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions be suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the compound to be used (i.e., the material for the hole injecting layer) and the crystal structure and the recombination structure of the target hole injecting layer.

Then, the light emitting layer is formed on the hole injecting layer formed above. A thin film of the light emitting material can be formed by using a light emitting material of the present invention in accordance with a process such as the vacuum vapor deposition process, the sputtering process, the spin coating process, or the casting process, and the formed thin film is used as the light emitting layer. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition of the hole injecting layer, although the conditions are different depending on the used compound.

Next, an electron injecting layer is formed on the light emitting layer formed above. Similarly to the hole injecting layer and the light emitting layer, in this case, it is preferable that the electron injecting layer be formed in accordance with the vacuum vapor deposition process since a uniform film must be obtained. The conditions of the vacuum vapor deposition can be selected in the same ranges as those described for the vacuum vapor deposition of the hole injecting layer and the light emitting layer.
When the vapor deposition process is used, the compound of the present invention can be deposited by vapor in combination with other materials, although the situation may be different depending on which layer in the light emitting zone or in the hole transporting zone includes the compound. When the spin coating process is used, the compound can be incorporated into the formed layer by using a mixture of the compound with other materials.
A cathode is formed on the electron injecting layer formed above in the last step, and an organic EL device can be obtained. The cathode is made of a metal and can be formed in accordance with the vacuum vapor deposition process or the sputtering process. It is preferable that the vacuum vapor deposition process be used in order to prevent damages on the lower organic layers during the formation of the film.
In the above-mentioned preparation of the organic EL device, it is preferable that the above-mentioned layers from the anode to the cathode be formed successively while the preparation system is kept in a vacuum after being evacuated once.

The method of forming the layers in the organic EL device of the present invention is not particularly limited. A conventionally known process such as the vacuum vapor deposition process and the spin coating process can be used. The organic thin film layer which is used in the organic EL device of the present invention and includes the amine-based compound of the present invention can be formed in accordance with a known process such as the vacuum vapor deposition process and the molecular beam epitaxy process (the MBE process) or, using a solution prepared by dissolving the compounds into a solvent, in accordance with a coating process such as the dipping process, the spin coating process, the casting process, the bar coating process, or the roll coating process.
The thickness of each layer in the organic thin film layer in the organic EL device of the present invention is not particularly limited. In general, a thickness in the range of several nanometers to 1 µm is preferable in order to prevent defects such as pin holes and improve efficiency.
The organic EL device which can be prepared as described above emits light when a direct voltage of 5 to 40 V is applied in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

Next, the present invention will be described in more detail by way of examples. However, the present invention is not limited to these examples at all.

### Synthesis Example 1 (synthesis of Compound 1)

A synthetic route for Compound 1 is shown below.

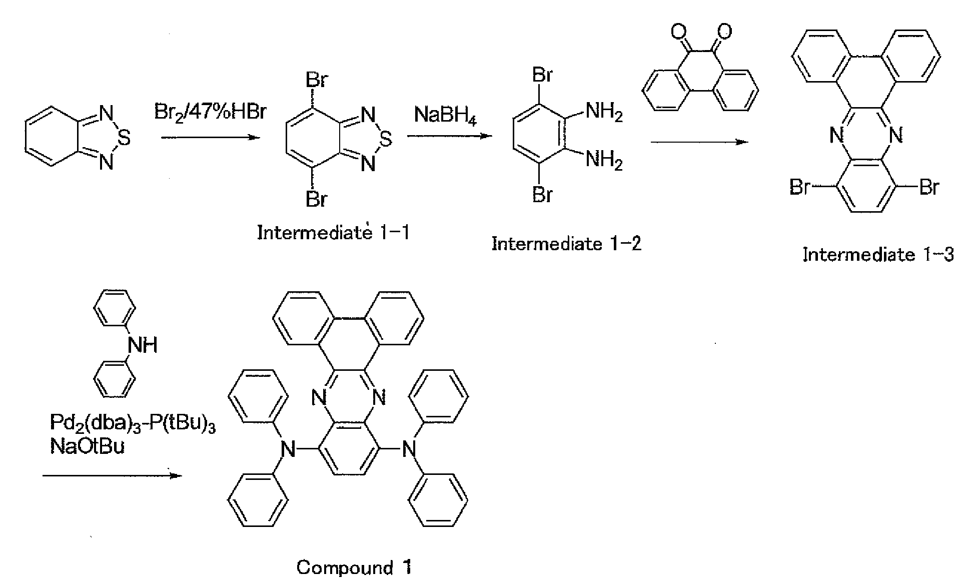

### (1) Synthesis of Intermediate 1-1

18.1 g (133 mmol) of benzothiadiazole were loaded into a 200-ml three-necked flask, and were dissolved in 42.9 ml of a 47% aqueous solution of HBr. 20 ml of bromine were dropped to the solution at room temperature over 20 minutes. Further, 13.3 ml of a 47% aqueous solution of HBr were added to the mixture, and the whole was refluxed for 24 hours. The temperature of the resultant was cooled to room temperature, and the resultant solid was dissolved in 700 ml of dichloromethane. After that, 400 ml of a saturated aqueous solution of sodium thiosulfate were added to the solution, and the whole was sufficiently extracted by using a separating funnel. Further, a dichloromethane layer was washed with 150 ml of distilled water 3 times, and was then dried with anhydrous sodium sulfate. Dichloromethane was concentrated to 200 ml, and the resultant was left at 4°C for 24 hours, whereby recrystallization was performed. The precipitated needle-like crystal was separated by filtration, whereby 28.2 g of a target compound were obtained (72% yield).

### (2) Synthesis of Intermediate 1-2

10.0 g (FW 293.96, 34 mmol) of Intermediate 1-1 and 400 ml of ethanol as a solvent were loaded into a 500-ml three-necked flask equipped with a reflux tube. Under ice cooling, 24.2 g (formula weight (FW) 37.83, 640 mmol) of sodium borohydride were added to the mixture, and the whole was sufficiently stirred for about 2 hours. When the generation of a gas (hydrogen sulfide) stopped, the temperature of the resultant was returned to room temperature, and the resultant was left overnight (about 14 hours). Next, the solvent was completely removed by distillation under reduced pressure, and about 400 ml of water was added to dissolve the remainder. The solution was left as it was overnight, whereby a target product was recrystallized (7.17 g). Next, the resultant was extracted with ethyl ether 3 times, washed with a saturated salt solution, dried with anhydrous sodium sulfate, filtered, and distilled to remove diethyl ether, whereby 8.05 g of a target compound were obtained. The total yield was 82%.

### (3) Synthesis of Intermediate 1-3

Intermediate 1-2 (FW287.90, 24.3mmol, 7.0g), 300mlofethanol as a solvent, and 5. 1 g (FW208, 24.3mmol) of 9,10-phenanthrenequinone were added to a 500-ml three-necked flask, and the whole was stirred for 8 hours. Recrystallization was repeated 5 times by using ethanol as a solvent, whereby a target product was obtained in a substantially quantitative manner.

### (4) Synthesis of Compound 1

Under an argon atmosphere, Intermediate 1-3 (FW 438, 5.7 mmol, 2.5 g), 2.3 g (FW 169, 13.7 mmol) of diphenylamine, 0.10 g (FW 916, 0.11 mmol) of Pd₂ (dba)₃ (where dba represents dibenzylildeneacetone), 1.4 g (FW 96, 14. 3 mmol) of t-butoxysodium, 20 ml of anhydrous toluene, and 36 mg (FW 202, 0.18 mmol) of P(tBu) (where tBu represents a t-butyl group) were added to a three-necked flask, and the whole was stirred at 80°C for 8 hours. The reaction liquid was purified by means of silica gel column chromatography, whereby 3 g of a purple solid as Compound 1 were obtained. The yield was 86%.
The results of measurement of the field desorption mass spectrum (FD-MS), ionization potential, and Tg (glass transition temperature) measured by differential scanning calorimetry (DSC) of the resultant product are shown below.
FD-MS: Calcd. for C₄₄H₃₀N₄ = 614 Found = 614
Ionization potential: 5.58 eV
Tg: 98°C

### Synthesis Example 2 (synthesis of Compound 2)

A synthetic route for Compound 2 is shown below.

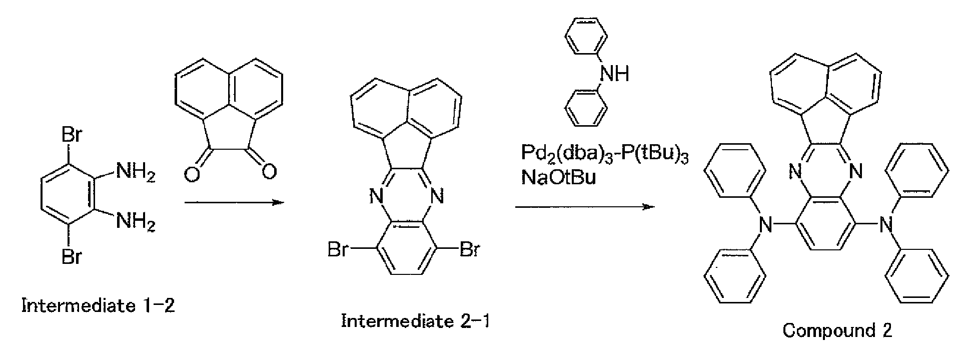

### (1) Synthesis of Intermediate 2-1

8.5 g of Intermediate 2-1 were obtained in a yield of 80% in the same manner as in the above item (3) of Synthesis Example 1 except that 4.4 g (FW 182, 24.3 mmol) of acenaphthenequinone were used instead of 5.1 g of 9,10-phenanthrenequinone.

### (2) Synthesis of Compound 2

3.0 g of an orange solid as Compound 2 were obtained in the same manner as in the above item (4) of Synthesis Example 1 except that Intermediate 2-1 (FW 438, 5.7 mmol, 2.3 g) was used instead of 2.5 g of Intermediate 1-3. The yield was 90%.
The results of measurement of the field desorption mass spectrum (FD-MS), ionization potential, and Tg (glass transition temperature) measured by differential scanning calorimetry (DSC) of the resultant product are shown below.
FD-MS: Calcd. for C₄₂H₂₈N₄ = 588 Found = 588
Ionization potential: 5.19 eV
Tg: A peak temperature was not detected.

Devices and measurement conditions adopted in the FD-MS measurement, DSC measurement, and ionization potential measurement of each of Synthesis Examples 1 and 2 are shown below.

| <FD-MS measurement> | | |
|---|---|---|
| Device: | HX 110 (manufactured by JEOL Ltd.) | |
| Conditions: | acceleration voltage 8 kV | |
| | scan range | m/z = 50 to 1,500 |
| | emitter | kind carbon |
| | emitter current | 0 mA → 2 mA/min → 40 mA (held for 10minutes) |

| <DSC measurement> | |
|---|---|
| Device: | Pyris 1 manufactured by PerkinElmer |
| Conditions: | (1) first heating 30°C → 260°C rate of temperature increase 10°C/min, all in a nitrogen atmosphere |
| | (2) 260°C held for 3 minutes |
| | (3) 260°C → - 50°C quenching 200°C/min |
| | (4) - 50°C held for 10 minutes |
| | (5) second heating - 50°C → 260°C rate of temperature increase 10°C/min |

| <Ionization potential measurement | |
|---|---|
| Device: | photoelectron spectrometer AC-1 (Rigaku Corporation) |
| | Conditions: powdered state, in the air |

### Example 1

A glass substrate with an ITO transparent electrode measuring 25 mm wide by 75 mm long by 1.1 mm thick (manufactured by GEOMATEC Co. , Ltd.) was subjected to ultrasonic cleaning in isopropyl alcohol for 5 minutes. After that, the substrate was subjected to UV ozone cleaning for 30 minutes. The glass substrate with the transparent electrode line after the washing was mounted on a substrate holder of a vacuum deposition device. First, Compound 1 was formed into a film having a thickness of 60 nm on the surface on the side where the transparent electrode line was formed to cover the transparent electrode. The Compound 1 functions as a hole injecting layer. Next, an N,N,N',N'-terakis(4-biphenyl)-4,4'-benzidine film (BPTPD film) having a thickness of 20 nm was formed on the Compound 1. The BPTPD film functions as a hole transporting layer. Further, Compound A having a thickness of 40 nm to be described below and doping Compound B at a deposition rate of 40:2 were simultaneously deposited on the BPTPD film. The film functions as a light emitting layer. Tris(hydroxyquinoline)aluminum (Alq to be described below) was formed into a film having a thickness of 10 nm on the resultant film. The Alq film functions as an electron transporting layer. After that, Li serving as a reducing dopant (Li source: manufactured by SAES Getters) and Alq were subjected to co-deposition. Thus, an Alq: Li film (having a thickness of 10 nm) was formed as an electron injecting layer (cathode). Metal Al was deposited from the vapor onto the Alq:Li film to form a metal cathode. Thus, an organic EL device was formed.
The resultant organic EL device was evaluated. As a result, the device had a current efficiency of 9.2 cd/A, and a half lifetime of 500 hours or longer for an initial luminance of 1,000 cd/m². In addition, the device had a maximum luminous wavelength of 473 nm.

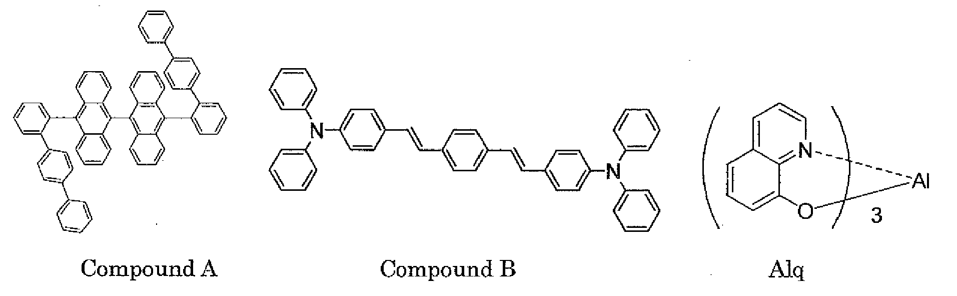

### Example 2

A device was produced in the same manner as in Example 1 except that Compound 2 was used instead of Compound 1. The resultant organic EL device was evaluated. As a result, the device had a current efficiency of 9.1 cd/A, and a half lifetime of 500 hours or more for an initial luminance of 1, 000 cd/m². Further, a maximum luminous wavelength was 473 nm.

### Comparative Example 1

A device was produced in the same manner as in Example 1 except that Comparative Compound 1 shown below was used instead of Compound 1. The resultant organic EL device was evaluated. As a result, the device had a current efficiency of 4.0 cd/A, and a half lifetime of 10 hours for an initial luminance of 1,000 cd/m².

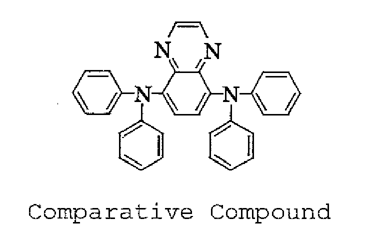

Comparative Compound 1 (FW 464) used in Comparative Example 1 reacted to decompose upon high-temperature heating (200 to 300°C), and multiple peaks including mass number 442 as the main component are observed by FD-MS measurement. A possible reason for the remarkably low light emission performance of Comparative Example 1 is the thermal decomposition of Comparative Compound 1 upon film formation during vapor deposition.
In order that the thermal decomposition may be suppressed to improve the light emission property of an organic EL device, the expansion of the conjugate structure of a quinoxaline structure and the introduction of a crosslinked structure are important as in the case of the amine-based compound of the present invention.

### INDUSTRIAL APPLICABILITY

As described above in detail, the novel amine-based compound of the present invention is suitable as a hole injecting material or hole transporting material for an electrophotographic photosensitive member or for an organic EL device. In addition, a thin film can be formed of the compound by an application method because the compound is excellent in solubility. In particular, an organic EL device using the amine-based compound brings physical properties, that is, a low ionization potential, large band gap energy, high injection efficiency, and a high mobility into balance in an excellent manner, has high heat resistance, and shows high current efficiency and a long lifetime while maintaining good luminance/voltage characteristics and good current density/voltage characteristics. Therefore, the device is extremely useful as an organic EL device having high practicality, and is suitable for an on-vehicle application where heat resistance is requested.

## Claims

1. An amine-based compound containing a quinoxaline ring and represented by the following general formula (1): 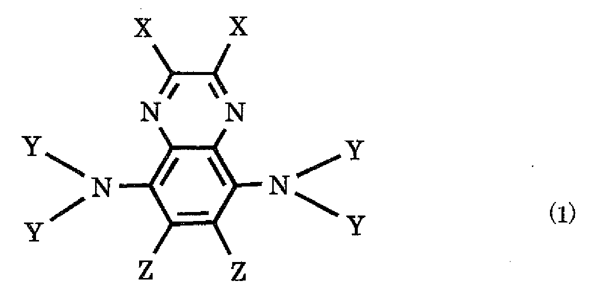 wherein:
X's each independently represent a hydrogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, the two X's may be identical to or different from each other, at least one X represents any one of the above-mentioned groups except a hydrogen atom, and the two X's crosslink with each other to form a cyclic structure;
Y's each independently represent a substituted or unsubstituted aryl group having 4 to 50 carbon atoms, or a substituted or unsubstituted heterocyclic group having 5 to 50 carbon atoms, and two Y's bonded to the same nitrogen atom may be identical to or different from each other, and may be bonded to and crosslink with each other; and
Z's each independently represent a hydrogen atom, a halogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, and the two Z's may be identical to or different from each other, and may be bonded to and crosslink with each other.

2. An amine-based compound containing a quinoxaline ring according to claim 1, which is represented by the following general formula (2): 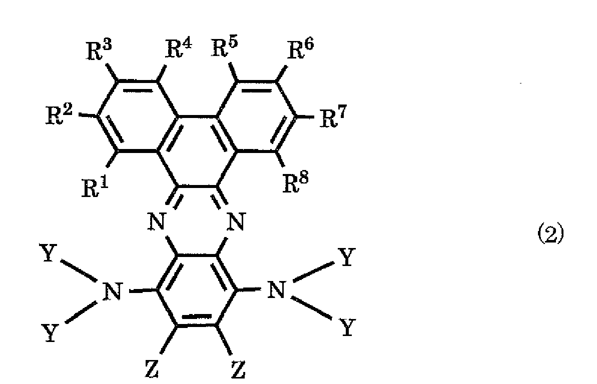 wherein R¹ to R⁸ each independently represent a hydrogen atom, a halogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, and may be identical to or different from one another, and two arbitrary adjacent groups of R¹ to R⁸ may crosslink with each other to form a cyclic structure, and Y's and Z's each have the same meaning as that described above.

3. An amine-based compound containing a quinoxaline ring according to claim 1, which is represented by the following general formula (3): 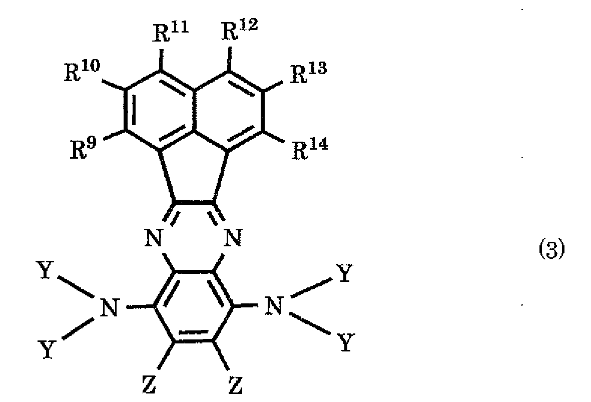 wherein R⁹ to R¹⁴ each independently represent a hydrogen atom, a halogen atom, a substituted or unsubstituted alkyl group having 1 to 50 carbon atoms, a substituted or unsubstituted alkoxy group having 1 to 50 carbon atoms, a substituted or unsubstituted aryloxy group having 4 to 50 carbon atoms, a substituted or unsubstituted thioalkoxy group having 1 to 50 carbon atoms, a substituted or unsubstituted thioaryloxy group having 4 to 50 carbon atoms, a substituted or unsubstituted amino group, a substituted or unsubstituted aryl group having 4 to 50 carbon atoms, a substituted or unsubstituted alkenyl group having 2 to 50 carbon atoms, a substituted or unsubstituted alkylcarbonyl group having 1 to 50 carbon atoms, or a substituted or unsubstituted arylcarbonyl group having 4 to 50 carbon atoms, and may be identical to or different from one another, and two arbitrary adjacent groups of R⁹ to R¹⁴ may crosslink with each other to form a cyclic structure, and Y's and Z's each have the same meaning as that described above.

4. An amine-based compound containing a quinoxaline ring according to any one of claims 1 to 3, wherein the amine-based compound comprises a hole injecting material or a hole transporting material.

5. An amine-based compound containing a quinoxaline ring according to any one of claims 1 to 3, wherein the amine-based compound comprises a hole injecting material for an organic electroluminescence device or a hole transporting material for an organic electroluminescence device.

6. An organic electroluminescence device, comprising an organic thin film layer composed of one or more layers including at least a light emitting layer, the organic thin film layer being sandwiched between a positive electrode and an anode, wherein at least one layer of the organic thin film layer contains the amine-based compound containing a quinoxaline ring according to any one of claims 1 to 3 alone or as a component of a mixture.

7. An organic electroluminescence device according to claim 6, wherein the organic thin film layer has at least one of a hole injecting layer and a hole transporting layer, and the at least one of the hole injecting layer and the hole transporting layer contains the amine-based compound alone or as a component of a mixture.

8. An organic electroluminescence device according to claim 6, wherein the light emitting layer emits blue-based light.

9. An organic electroluminescence device according to claim 6, wherein the light emitting layer contains an anthracene derivative as a host material.

10. An organic electroluminescence device according to claim 8 or 9, wherein the light emitting layer contains an amine derivative as a host material or as a dopant.
